# EUROPEAN PATENT SPECIFICATION

(11) **EP 3 542 860 B1**
(45) Date of publication and mention of the grant of the patent: **09.06.2021**
(21) Application number: 18213719.0
(22) Date of filing: 12.06.2015
(51) Int. Cl.: A61N 7/00

(54) **SYSTEM FOR FAST ULTRASOUND TREATMENT**
SYSTEM ZUR SCHNELLEN ULTRASCHALLBEHANDLUNG
SYSTÈME DE TRAITEMENT RAPIDE PAR ULTRASONS

(30) Priority: 13.06.2014 US 201462012266 P
(43) Date of publication of application: 25.09.2019
(62) Divisional of application: 15730663.0
(73) Proprietor: Guided Therapy Systems, L.L.C., Mesa, Arizona 85202 (US)
(72) Inventor: Barthe, Peter G., Phoenix, AZ 85048 (US); Slayton, Michael H., Phoenix, AZ 85018 (US)
(74) Representative: Maiwald Patent- und Rechtsanwaltsgesellschaft mbH

(56) References cited:
- US-A1- 2010 049 098
- US-A1- 2012 029 353

## Description

### BACKGROUND

Current ultrasound methods for treatment of a targeted media, including live tissue and soft tissue, are limited to a small treatment area. This is due to limitations in ultrasound technology, which limits the speed of treatment In this context, reference is made to documents US2010/0049098 A1 and US2012/0029353 A1.

### SUMMARY

The present disclosure overcomes the aforementioned drawbacks by presenting systems and methods for treating an area using ultrasound with increased speed and greater efficiency. The invention is defined by the appended claims.

In one aspect, this disclosure provides a treatment device for delivering an energy into a region of interest at or beneath a target surface. The treatment device can include a housing, a transducer module, and a control module. The transducer module can be coupled to the housing. The transducer module can include an energy source configure for the delivery of the energy. The transducer module can include a transducer module wall that includes a curved surface having a non-zero radius of curvature. The treatment device can be movable relative to the target surface while retaining contact between the curved surface and the target surface. The curved surface can be configure to retain coupling between the energy source and the region of interest upon a change in an angle of incidence of the energy relative to the target surface of at least 1 degree. The control module can be configure to control the energy source.

In another aspect, this disclosure provides a treatment device, which is configured for treating a first location and a second location within a region of interest at or below a target surface by delivery of an energy. The treatment device is configured to perform a method which can include: a) transmitting the energy, provided by an energy source of a treatment device, into the first location, the energy transmitted through a transducer module wall including a curved surface having a non-zero radius of curvature; b) monitoring, using a position sensor of the treatment device, a position of the treatment device relative to the target surface or a speed of the treatment device relative to the target surface The treatment device can be moved relative to the target surface while retaining contact between the curved surface and the target surface. The treatment device can further be configured for transmitting the energy, provided by the energy source of the treatment device, into the second location based on the monitoring of step b), the energy transmitted through the transducer module wall including the curved surface.

In yet another aspect, this disclosure provides a treatment device for delivering an energy into a region of interest at or beneath a target surface. The treatment device includes a housing, a transducer module, and a control module. The transducer module is coupled to the housing. The transducer module includes an energy source configure for the delivery of the energy. The energy can pass through an acoustic window prior to the delivery into the region of interest. The acoustic window can have a non-zero radius of curvature. The treatment device can be movable relative to the target surface while retaining contact between the acoustic window and the target surface. The acoustic window can be configured to retain coupling between the energy source and the region of interest upon a change in an angle of incidence of the energy relative to the target surface of at least 1 degree. The control module is configured to control the energy source.

The foregoing and other aspects and advantages of the invention will appear from the following description. In the description, reference is made to the accompanying drawings which form a part hereof, and in which there is shown by way of illustration a preferred embodiment of the invention. Such embodiment does not necessarily represent the full scope of the invention, however, and reference is made therefore to the claims and herein for interpreting the scope of the invention.

The present disclosure relates to the following embodiments:
According to the invention, a treatment device for delivering an energy into a region of interest at or beneath a target surface is provided. The treatment device comprises: a housing; a transducer module coupled to the housing, the transducer module comprising an energy source configured for delivery of the energy. The transducer module may have a transducer module wall that includes a curved surface having a non-zero radius of curvature, the treatment device movable relative to the target surface while retaining contact between the curved surface and the target surface, the curved surface configured to retain coupling between the energy source and the region of interest upon a change in an angle of incidence of the energy relative to the target surface of at least 1 degree. According to the invention, the treatment device further includes a control module configured to control the energy source.

According to an embodiment, the transducer module is sealed and contains a coupling medium to facilitate coupling of the energy source with the region of interest.

According to an embodiment, the energy source is at least one ultrasound transducer configured to controllably direct acoustic energy into the region of interest.

According to an embodiment, the energy source comprises at least one ultrasound transducer configured to controllably direct acoustic energy into the region of interest and a second energy source configured to provide a second non-acoustic energy to the region of interest.

According to an embodiment, the treatment device further comprising a contact sensor in communication with the control module and configured to determine when the energy source is coupled to the region of interest.

According to an embodiment, the control module terminates the delivery of energy if the contact sensor determines that the energy source is uncoupled to the region of interest.

According to an embodiment, the contact sensor comprises a hall detector, an optical detector, an acoustic impedance detector, a conductive detector, a piezo electric detector, a mechanical detector, a magnetic detector, a capacitive detector, or a combination thereof.

According to an embodiment, the control module terminates the delivery of energy if the position of the treatment device is outside a pre-defined area of treatment or if the speed of the treatment device is greater than an upper threshold.

According to an embodiment, the device further comprising an axle positioned through a center axis of the rolling member.

According to the invention, the energy source is an acoustic energy source. Additionally, the energy source may be a photon-based energy source, a radio-frequency energy source and/or a microwave energy source.

According to an embodiment, the energy is an acoustic energy. Additionally, the energy may be a photon-based energy, a radio-frequency energy, and/or a microwave energy.

According to an embodiment, the control module terminates delivery of the energy if the contact sensor determines that the energy source is uncoupled from the region of interest.

According to an embodiment, the energy source is configured to deliver the energy through an acoustic window having a thickness of about one half wavelength of the energy or about a simple multiple of one half wavelength of the energy.

According to an embodiment, the energy is reflected by the acoustic window when the energy source is uncoupled from the region of interest, which signals the control module to terminate the delivery of the energy.

According to an embodiment, the treatment device further comprising a rolling member coupled to the housing, the treatment device movable relative to the target surface by rotating the rolling member along the target surface in a rotational direction while retaining coupling between the energy source and the region of interest.

According to an embodiment, the rolling member is disposed outside an emission path of the energy from the energy source.

According to the invention, the treatment device further comprises a position sensor in communication with the control module and configured to transmit a position signal representative of a position of the treatment device to the control module. Additionally, the position sensor may be configured to transmit a speed signal representative of a speed of the treatment device to the control module. The control module may be configured to control the energy source based on the position signal or the speed signal.

According to a further embodiment, the treatment device is further configured to perform a method of treating a first location and a second location within a region of interest at or beneath a target surface by delivery of an energy. The method comprises: a) transmitting the energy, provided by an energy source of a treatment device, into the first location, the energy transmitted through a transducer module wall including a curved surface having a non-zero radius of curvature; b) monitoring, using a position sensor of the treatment device, a position of the treatment device relative to the target surface or a speed of the treatment device relative to the target surface. The treatment device is movable relative to the target surface while retaining contact between the curved surface and the target surface. The treatment device is further configured for transmitting the energy, provided by the energy source of the treatment device, into the second location based on the monitoring of step b), the energy transmitted through the transducer module wall including the curved surface.

According to a further embodiment, the treatment device is further configured for sensing, using a contact sensor of the treatment device, a coupling between the energy source and the region of interest.

According to a further embodiment, the treatment device is further configured so that the transmitting of the energy into the second location comprises either: terminating the energy transmission if the coupling between the energy source and the region of interest is interrupted; or transmitting the energy, provided by the energy source, into the second location if the coupling between the energy source and the region of interest is uninterrupted.

According to a further embodiment, the transmitted energy does not contact a rolling member of the treatment device.

According to a further embodiment, the transmitting of the energy into the first and second location is directed through the transducer module wall having a thickness of about one half wavelength of the energy or about a simple multiples of one half wavelength of the energy.

According to the invention, a treatment device for delivering an energy into a region of interest at or beneath a target surface is provided, wherein the treatment device comprises: a housing; a transducer module coupled to the housing, the transducer module comprising an energy source configured for delivery of the energy. According to an embodiment, the energy is passing through an acoustic window prior to the delivery into the region of interest, the acoustic window having a thickness equal to one half wavelength of the energy or a simple multiple of one half wavelength of the energy, the acoustic window having a non-zero radius of curvature, the treatment device movable relative to the target surface while retaining contact between the acoustic window and the target surface, the acoustic window configured to retain coupling between the energy source and the region of interest upon a change in an angle of incidence of the energy relative to the target surface of at least 1 degree. According to the invention, the control module configured to control the energy source.

According to a further embodiment, the treatment device further comprising a rolling member coupled to the housing, the treatment device movable relative to the target surface by rotating the rolling member along the target surface in a rotational direction while retaining coupling between the energy source and the region of interest.

According to a further embodiment, the rolling member is disposed outside an emission path of the energy from the energy source.

According to a further embodiment, the treatment device further comprises a contact sensor in communication with the control module and configured to determine when the energy source is coupled to the region of interest by identifying energy reflected back toward the energy source by the acoustic window.

### BRIEF DESCRIPTION OF THE DRAWINGS

Fig. 1 is a perspective view illustrating an exemplary treatment device, according to one aspect of the present disclosure.
Fig. 2 is a cross-sectional view along the line A-A of Fig. 1, according to one aspect of the present disclosure.
Fig. 3 is a cross-sectional view along the line B-B of Fig. 1, according to one aspect of the present disclosure.
Fig. 4 is a cross-sectional view along the line C-C of Fig. 3, according to one aspect of the present disclosure.
Fig. 5 is a cross-sectional view illustrating an exemplary treatment device, according to one aspect of the present disclosure.
Fig. 6 is a cross-sectional view illustrating an exemplary treatment device, according to one aspect of the present disclosure.
Fig. 7 is a cross-sectional view illustrating an exemplary treatment device, according to one aspect of the present disclosure.
Fig. 8 is a cross-sectional view illustrating an exemplary treatment device, according to one aspect of the present disclosure.
Fig. 9 is a view from beneath an exemplary treatment device, according to one aspect of the present disclosure.
Fig. 10 is a cross-sectional view along line D-D of Fig. 9, according to one aspect of the present disclosure.
Fig. 11 is a view from beneath an exemplary treatment device, according to one aspect of the present disclosure.
Fig. 12 is a cross-sectional view along line E-E of Fig. 11, according to one aspect of the present disclosure.
Fig. 13 is a cross-sectional view of an exemplary treatment device, according to one aspect of the present disclosure.
Fig. 14 is a view from beneath an exemplary treatment device, according to one aspect of the present disclosure.
Fig. 15 is a view from beneath an exemplary treatment device, according to one aspect of the present disclosure.
Fig. 16 is a view from beneath an exemplary treatment device, according to one aspect of the present disclosure.
Fig. 17 is a cross-sectional view along the line of an exemplary treatment device, according to one aspect of the present disclosure.
Fig. 18 is a partial cross-section of an exemplary treatment device illustrating a lensing effect, according to one aspect of the present disclosure.
Fig. 19A is a graph of a acoustic power transmission factor versus frequency for a first transducer wall thickness.
Fig. 19B is a graph of a acoustic power transmission factor versus frequency for a second transducer wall thickness.
Fig. 19C is a graph of a acoustic power transmission factor versus frequency for a third transducer wall thickness.
Fig. 20 is a graph illustrating resistance over time for an exemplary treatment device coupled to tissue, according to one aspect of the present disclosure.
Fig. 21 is a graph illustrating resistance over time for an exemplary treatment device not coupled to tissue, according to one aspect of the present disclosure.
Fig. 22 is a matrix diagram illustrating a variety of transducer and lens configurations, according to one aspect of the present disclosure.
Fig. 23 is a flowchart illustrating exemplary methods, according to one aspect of the present disclosure.
Fig. 24 is an illustration of the treatment device described in Example 1.
Fig. 25 is an photograph of the treatment device described in Example 1 applying treatment lines to a thermally sensitive plastic.

### DETAILED DESCRIPTION

Before the present invention is described in further detail, it is to be understood that the invention is not limited to the particular embodiments described. It is also to be understood that the terminology used herein is for the purpose of describing particular embodiments only, and is not intended to be limiting. The scope of the present invention will be limited only by the claims. As used herein, the singular forms "a", "an", and "the" include plural embodiments unless the context clearly dictates otherwise.

Specific structures, devices, and methods relating to improved ultrasound treatment efficiency and operation are disclosed. It should be apparent to those skilled in the art that many additional modifications beside those already described are possible without departing from the inventive concepts. In interpreting this disclosure, all terms should be interpreted in the broadest possible manner consistent with the context. Variations of the term "comprising" should be interpreted as referring to elements, components, or steps in a non-exclusive manner, so the referenced elements, components, or steps may be combined with other elements, components, or steps that are not expressly referenced. Embodiments referenced as "comprising" certain elements are also contemplated as "consisting essentially of" and "consisting of" those elements.

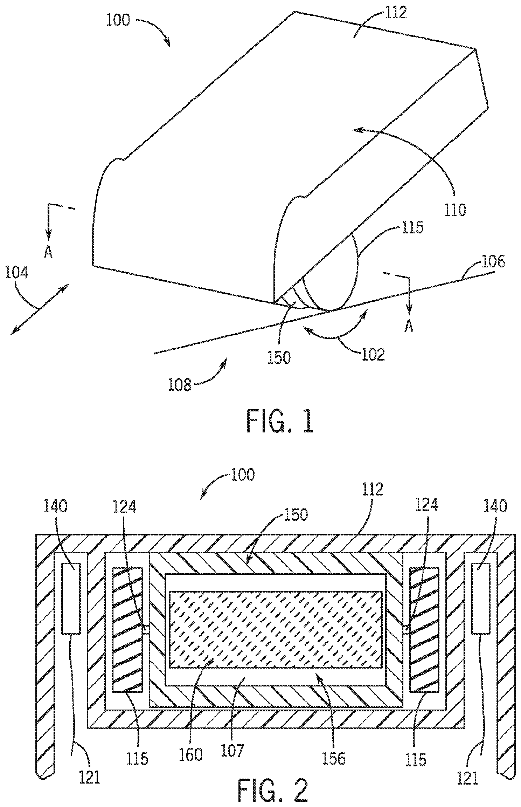

With reference to Fig. 1, a treatment device 100 is illustrated, in accordance with the present disclosure. The treatment device 100 can include a control module 110, a transducer module 150, and one or more rolling members 115. The transducer module 150 can be configured to contact a target surface 106. The treatment device 100 can be configured to treat a region of interest ("ROI") 108, such as, for example, a ROI 108 at or beneath the target surface 106. The treatment device 100 can include a housing 112. The housing 112 can be configured to encase the control module 110. The control module 110 can also be located outside the housing 112 or can be partially located inside the housing 112 and partially located outside the housing 112.. The treatment device 100 can move in a forward direction 104, which can rotate the rolling member 115 in a rotational direction. The treatment device 100 can move in either direction, forward or back, and can move in an arcing motion or essentially a straight line. The treatment device 100 can move in response to an external force, such as a user or a robotic implement pushing or pulling the treatment device 100, or an internal force, such as a drive motor coupled to the one or more rolling members 115.

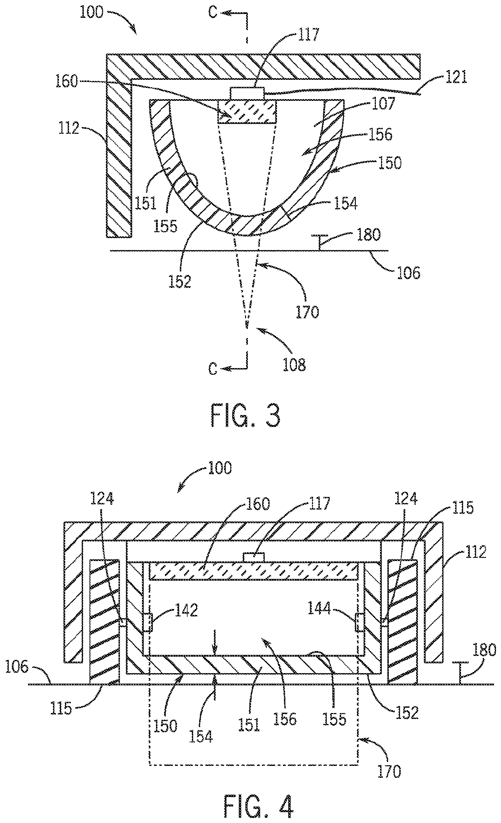

A cross-sectional view of the treatment device 100 along line A-A of Fig. 1 is illustrated in Fig. 2 and a cross-sectional view of the treatment device 100 along line B-B of Fig. 1 is illustrated in Fig. 3. The treatment device 100 can include a transducer module 150, which can include at least one energy source 160. One or more axles 124 can be positioned through the center axis of the one or more rolling members 115 and can connect the one or more rolling members 115 to the housing 112. In certain aspects, the transducer module 150 is connected to the housing 112 and the one or more axles 124 can connect the one or more rolling members 115 to the transducer module 150. The transducer module 150 can be coupled to the housing 112 and can be configured to remain coupled to the treatment surface 106 while the one or more rolling members 115 rotate in a rotational direction 102 along the surface 106. The transducer module 150 can be sealed to form an internal volume 156 that can contain a coupling medium 107 configured to facilitate coupling of the energy source 160 with tissue in the ROI 108. In some aspects, the energy source 160 is at least one ultrasound transducer configured to controllably direct acoustic energy 170 into the ROI 108.

Referring to Fig. 2, the treatment device 100 can include one or more position sensors 140. The position sensors 140 can be provided in electrical communication with the control module 110 by way of a wired connection 121, as illustrated, or in the form of a wireless connection known to those having ordinary skill in the art. In aspects of the treatment device 100 having two or more position sensors 140, such as the treatment device 100 illustrated in Fig. 2, the two or more position sensors 140 can work in concert to provide information relating to the orientation of the treatment device 100. In certain aspects, one position sensor 140 can correspond to one rolling member 124, thus providing information on the position of the corresponding rolling member 124.

Referring to Fig. 3, the treatment device 100 can include an interface 117 for coupling the transducer module 150 to the housing 112. The interface 117 can be configured to allow the transducer module 150 to be detachable from the housing 112. In certain aspects, the interface 117 establishes an electrical communication between the transducer module 150 and the control module 110 when the transducer module 150 is connected to the housing 112 and terminates the electrical communication when the transducer module 150 is detached from the housing 112. The electrical communication can be in the form of a wired connection 121, as illustrated, or in the form of a wireless connection known to those having ordinary skill in the art.

The transducer module 150 can include a transducer module wall 151 having an outer surface 152 and an inner surface 155. The transducer module wall 151 has a thickness 154, which is a distance measured perpendicularly from the outer surface 152 to the inner surface 155. In various configurations, the thickness 154 can be configured to provide various physical attributes to ultrasound energy 170, as further discussed herein. The transducer module 150 can be configured to have an internal volume 156, which is bordered or bounded by the inner surface 155. The internal volume 156 can be filled with a coupling medium 107, as discussed herein.

The transducer module wall 151 can include a curved surface having a non-zero radius of curvature. In certain aspects, the treatment device 100 can be movable relative to the target surface 106 while retaining contact between the curved surface and the target surface. As used herein, "retaining contact" shall refer to retaining direct contact, as well as instances where a thin layer of coupling medium 107 is located between the curved surface and the target surface 106. In certain aspects, the curved surface can be configured to retain coupling between the energy source 160 and the region of interest 108 upon a change in an angle of incidence of the energy 170 relative to the target surface 106 of at least 1 degree, at least 2 degrees, at least 3 degrees, at least 5 degrees, at least 7.5 degrees, at least 10 degrees, at least 15 degrees, at least 20 degrees, at least 25 degrees, or at least 30 degrees.

In certain aspects, the curved surface is an acoustic window.

In certain aspects, referring to Fig. 3, the treatment device 100 can be configured such that there is an offset 180 between the outer surface 152 of the transducer module wall 151 and the treatment surface 106. In certain aspects, the offset 180 can be filled with a coupling medium 107.

A cross-sectional view of the treatment device 100 along line C-C of Fig. 3 is illustrated in Fig. 4. The transducer module 150 can include a transducer module wall 151, which has an outer surface 152 and an inner surface 155. The transducer module wall 151 can have a thickness 154, which is a distance measured perpendicularly from the outer surface 152 to the inner surface 155. In various configurations, the transducer module wall 151 can be configured to provide various physical attributes to the energy 170, as further discussed herein. The transducer module 150 can be configured to have an internal volume 156, which is bordered or bounded by the inner surface 155. The internal volume 156 can be filled with a coupling medium 107, as discussed herein. The transducer module 150 can include an energy source, which can include an ultrasound transducer. The ultrasound transducer can include a single face or a number of faces. In certain aspects, the energy source can be an array of transduction elements.

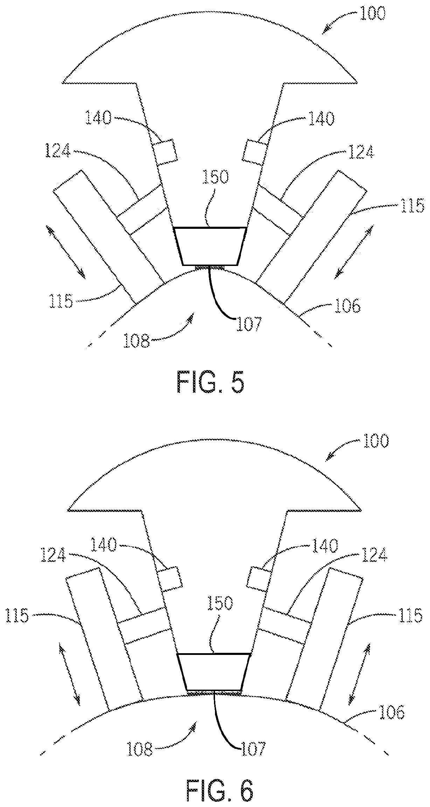

Referring to Fig. 5 and 6, a treatment device 100 can include a housing 112, a control module, 110, a transducer module 150, and at least two rolling members 115. The rolling members 115 can each be coupled to the housing 112 by way of an axle 124. The axles 124 can be movable relative to the housing 112. The treatment device 100 can include position sensors 140. Each position sensor 140 can be located and configured to monitor the position and/or speed of an associated rolling member 115.

In certain aspects, the axles 124 can be sensitive to mechanical pressure on the rolling members 115. For example, an axle 124 can be configured to move upward into the housing 112 in response to an increase in mechanical pressure on the associated rolling member 115. Similarly, an axle 124 can be configured to move downward toward the surface 106 in response to a decrease in mechanical pressure on the associated rolling member 115. The axles 124 can be spring-loaded to move up and down along the housing body to compensate for variations in the relative height of the surface 106. In certain aspects, the axles 124 can be configure to move in a direction that is normal to the surface 106.

In certain aspects, the treatment device 100 can include first rolling member 115 at a first end of the transducer module 150 and a second rolling member 115 at a second end of the transducer module 150, each mounted on a movable axle 124. The movable axles 124 can be utilized to provide any desired configuration of the transducer module 150 relative to the ROI 108. The first and second rolling members 115 and the first and second axles 124 can have their movements coordinated to move the transducer module 150 in one or more of six degrees of motion. For example, the first and second rolling members 115 and the first and second axles 124 can be configured to compensate for any of yaw, pitch, roll, or combinations thereof as the treatment device 100 is moved across a target surface 106.

Fig. 5 shows use of the treatment device 100 on a surface 106 that has a steeper slope, whereas Fig. 6 shows use of the treatment device 100 on a flatter surface 106. In one exemplary application for treatment of an Achilles tendon, the treatment device 100 can be initially positioned as shown in Fig. 5 at the narrow base of the tendon next to the foot. As energy 170 is delivered into the region of interest 108 (in this case, the tendon), the treatment device 100 is rolled up the tendon toward the calf, where the treatment device 100 is positioned as shown in Fig. 6 at the wider portion of the calf. In certain applications, the outer surface 152 of the transducer module 150 can have a convex shape to wrap around a concave surface 106 and maximize contact between the outer surface 151 and the concave surface 106.

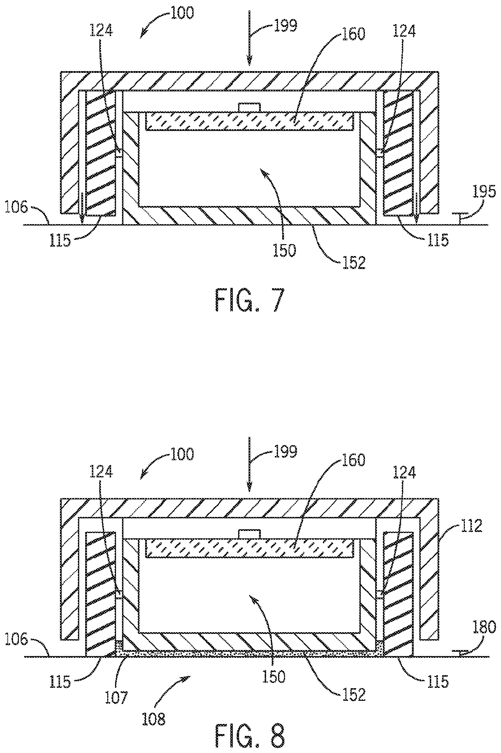

Referring to Figs. 7 and 8, a treatment device 100 is illustrated where pressure sensitive axles 124 are utilized, such that a force 199 can be applied to the treatment device 100 in order for the rolling members 115 or the transducer module 150 to be moved downward toward the target surface 106. In certain applications, in a neutral position, the transducer module 150 can rest on the target surface 106 and the rolling members 115 can be raised to form a wheel gap 195 between the rolling members 115 and the target surface 106. In these applications, application of the force 199 moves the rolling members 115 downward to engage the target surface 106. The pressure 199 can be calibrated to provide an offset 180, as desired. Moreover, the pressure 199 can be calibrated to provide a proper standoff distance and to ensure precise delivery of the energy 170 to a desired depth in the ROI 108.

In certain applications, in a neutral position, the rolling members 115 can rest on the target surface 106 and the transducer module 150 can be raised to form a transducer gap (not illustrated) between the transducer module 150 and the target surface 106. In these applications, application of force 199 moves the transducer module 150 downward to engage or couple to the target surface 106 and/or to move near to the target surface 106 to provide the offset 108.

In certain aspects, the outer surface 151 can be concave, and when force 199 is applied, the concave outer surface 151 can couple to the target surface 106.

In certain aspects, monitoring the force 199 can be used in a treatment function, as described herein. For example, a closed-loop feedback can communicate to the control module 110 a signal indicating if the force 199 exceeds a minimum force. If the minimum force is not being applied, the treatment function can send a stop treatment signal to the control module 110 to terminate the application of energy to the ROI 108.

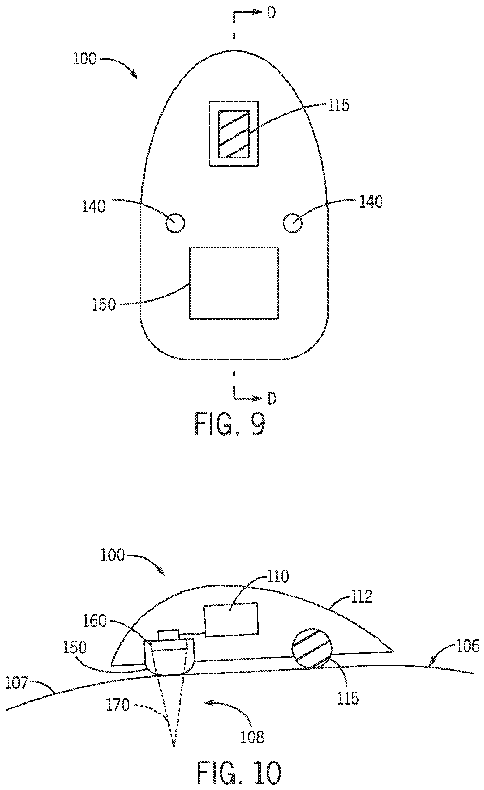

Referring to Figs. 9 and 10, a treatment device 100 is illustrated where one or more rolling members 115 and one or more transducer modules 150 combine to provide stable engagement with the contact surface. Fig. 10 is a cross-section of the treatment device 100 of Fig. 9, along the line D-D. The one or more rolling members 115 and the one or more transducer modules 150 can be sized and shaped appropriately to provide stability to the treatment device 100. The one or more rolling members 115 and the one or more transducer modules 150 can be positioned relative to one another such that a rolling member 115 is located in front, on a side, or behind a transducer module 150 as the treatment device 100 is moved.

Referring to Fig. 9, a treatment device 100 is illustrated where position sensors 140 are configured to enable determination of the position and/or orientation of the treatment device 100 independent of any corresponding rolling member 115.

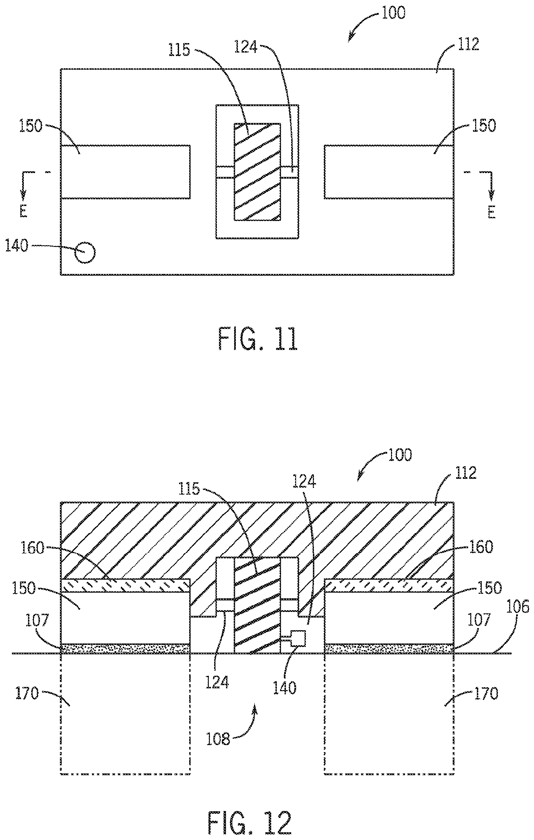

Referring to Figs. 11-14, various configurations of a treatment device 100 are illustrated, where a rolling member 115 is located between two transducer modules 150. In certain aspects, the treatment device can include two transducer modules 150, a controller 110, one rolling member 115, and at least one position sensor 140 that is associated with the one rolling member 115 (as illustrated in Fig. 12). In certain aspects, as illustrated in Fig. 11, the treatment device 100 can include a position sensor 140 that is not associated with the one rolling member 115. Fig. 11 represents a view of the treatment device 100 from the perspective of the treatment surface (not shown) looking upward to the treatment device 100. Fig. 12 is a cross-sectional view along the line E-E of Fig. 11. As shown in Fig. 12, the transducer modules 150 and corresponding energy sources 160 can be configured to deliver the energy 170 directly below the transducer modules 150. The energy 170 can be directed to the area below the transducer modules 150 in a strongly focused, weakly focused, defocused, or unfocused form.

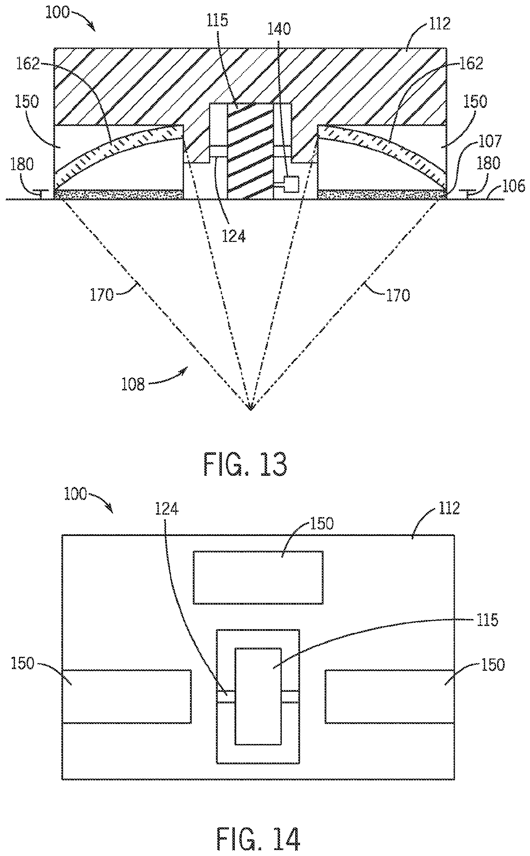

Referring to Fig. 13, the treatment device 100 can include two transducer modules 150 having transducers 162 that are configured to focus the energy 170 to a center area of the ROI 108, located beneath the rolling member 115. The energy 170 can similarly be directed to the center area in a strongly focused, weakly focused, defocused, or unfocused form.

Referring to Fig. 14, the treatment device 100 can include three transducer modules 150, a controller 110, and one rolling member 115.

The aspects of the invention shown in Figs. 11-14 can be combined with other aspects of the disclosure as described herein, such as a pressure-sensitive axle 124, unless expressly ruled out by the one-wheel nature of the treatment devices 100 illustrated therein.

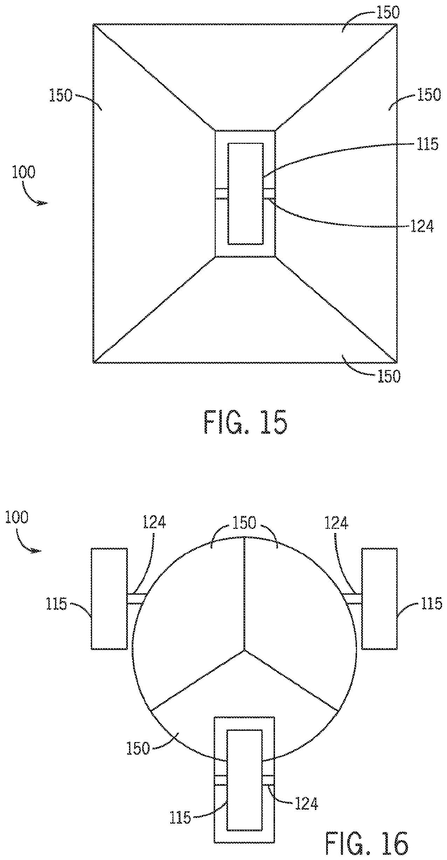

Referring to Fig. 15, a treatment device 100 including four transducer modules 150 is illustrated. In the illustrated aspect, the transducer modules 150 are adapted to fit within a square or a rectangle. In the illustrated aspect, the treatment device 100 includes one rolling member 115 positioned between the four transducer modules 150. The rolling member 115 can be supported by an axle 124. The rolling member 115 and axle 124, and/or the four transducer modules 150 can be configured to be pressure-sensitive as described elsewhere herein to move relative to the target surface 106 when a force is applied to the treatment device 100.

Referring to Fig. 16, a treatment device 100 including three transducer modules 150 is illustrated. In the illustrated aspect, the transducer modules 150 are adapted to fit within a circular- or dome-shaped configuration. In the illustrated aspect, the treatment device 100 can include three rolling members 115 and three associated axles 124. The rolling members 115 and axles 124, and/or the three transducer modules 150 can be configured to be pressure-sensitive as described elsewhere herein to move relative to the target surface 106 when a force is applied to the treatment device 100.

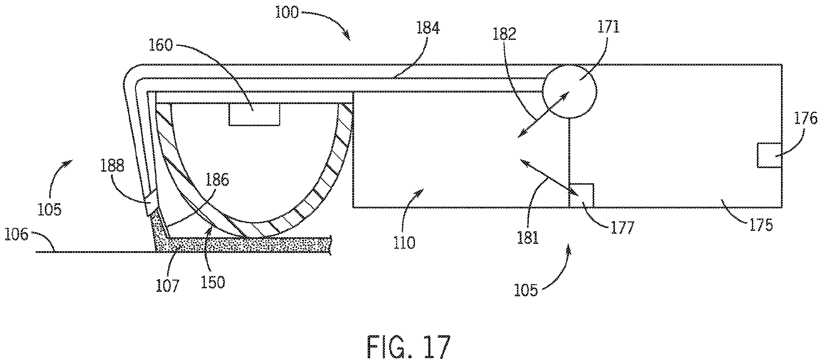

Referring to Fig. 17, an example of the treatment device 100 is illustrated as a cross-sectional view of, for example, the system of Fig. 1. As discussed herein, the treatment device 100 can include a control module 110, a coupling medium application device 105, and a transducer module 150. In some configurations, the coupling medium application device 105 can include a coupling medium reservoir 175 that is in communication with a pump 171, an application tube 184, and a nozzle 188. In addition, the coupling medium application device 105 can include a fill port 176 and level sensor 177. As the treatment device 100 moves, the movement of the transducer module 150 is communicated to the control module 110, as discussed herein. The pump 171 is configured for communication 182 with the control module 110. As the movement of the transducer module 150 is received by the control module 110, calculations are made within the control module 110 and are sent via communication 182 to instruct the pump 171 to meter out a desired amount of coupling medium 107 from the coupling medium reservoir 177 into the application tube 184. The desired amount of coupling medium 107 moves through the application tube 184 through the nozzle 188 and onto the target surface 106 to provide coupling between the transducer module 150 and the ROI 108. The nozzle 188 can include a sweep 186 configured to apply an even coat of the coupling medium 107 onto the surface 106.

In some applications, the nozzle 188 can include a valve configured to open for application of the coupling medium 107 onto the target surface 106 or to close when the transducer module 150 has stopped. The level sensor 177 can be configured for communication 181 with the control module 110. In some applications, the level sensor 177 provides communication 182 to control module 110, which indicates a level of coupling medium 107 remaining in the reservoir tank 175 and, for example, when the reservoir tank 175 is empty. Upon receiving an empty communication, the control module 110 can stop operation of transducer device 100. In some aspects, upon receiving level communications indicating empty or near empty conditions, the control module 110 can communicate to user a warning signal such as a visual indicator and/or an audio signal. The coupling medium reservoir 175 can be refilled via the fill port 176.

The coupling medium 107 can be any such materials, gels, medicaments, as discussed herein, or are known to those skilled in the art now or at any time in the future. In some configurations, the coupling medium application device 105 includes a bias member, which is employed to push the coupling medium 107 onto transducer assembly 150 and/or keep the coupling medium 107 in contact with the transducer assembly 150. The coupling medium application device 105 can be adapted to be employed with any of the configurations described herein. In addition, the coupling medium application device 105 can be adapted to any of the configurations of the transducer module 150.

The control module 110 can be configured to receive at least one communication and control a distribution of the acoustic energy 170 transmitted by the energy source 160, such as, for example, an acoustic transducer. The control module 110 can be configured to receive a treatment start signal and a treatment stop signal. The control module 110 can be programmed to provide treatment to a ROI 108 for a desired outcome. The control module 110 can initiate and run a treatment program (treatment function), which can include the control of spatial parameters and/or temporal parameters of the energy source 160, to provide programmed distribution energy 170 in the ROI 108. The control module 110 can be configured to receive feedback from one or more sensors and/or detectors, and the control module 110 can terminate the treatment program based on the feedback. For example, it can terminate the delivery of energy if the position of the treatment device is outside a pre-defined area of treatment. Other examples will be provided hereinafter.

The interface 117 can include an electronic interface between the control module 110 and the transducer module 150. The interface 117 can include a thin film electrode configured to provide an electronic interface between the control module 110 and the transducer module 150.

The treatment device 100 can include a position sensor 140 in communication with the control module 110 and configured to monitor a position of the transducer module 150 or any other portion of the treatment device 100, or to calculate a speed of the rotation in the rotational direction 102 of the rolling member 124. In some configurations, the rolling member 124 or the transducer module 150 can include the position sensor 140. The position sensor 140 can be integrated into the rolling member 124 or attached to the rolling member 124. The position sensor 140 can be configured to measure movement of the rolling member 124 or the transducer module 150.
The position sensor 140 calculates a distance traveled (for example a delta distance or a distance change) along the target surface 106. For example, the position sensor 140 may determine a speed of movement of the transducer module 150 and determine if the speed is within programmed limitations for providing treatment. For example if the speed is beyond a desired or effective speed, the position sensor 140 can provide a signal indicating the speed or an alert or other information to indicate the over-speed condition. The over-speed condition can be provided to a user and/or to a control module 110 to control, adjust, or stop emitting the therapeutic ultrasound energy 170.

The position sensor 140 is programmed to determine a distance between pulses of therapeutic ultrasound energy 170 to create a plurality of treatment zones. The treatment zones may be evenly spaced or can be disposed in any spatial configuration in one-, two-, or three-dimensions. As the transducer module 150 is moved in the direction 104, the position sensor 140 determines a distance, regardless of a speed that the transducer module 150 is moved, at which a pulse of the therapeutic ultrasound energy 170 is to be emitted into ROI 108, as programmed into the control module 110. In some configurations, the transducer module 150 is triggered automatically via a timer and in combination with the position sensor 140 to assure movement of the transducer module 150.

The control module 110 can terminate the distribution of the acoustic energy 170 into the ROI 108, if the speed of the rotation in the rotational direction 102 of the rolling member 115 is below a minimum speed. The control module 110 can terminate the distribution of the acoustic energy 170 into the ROI 108, if the speed of the rotation in the rotational direction 102 of the rolling member 115 is above a desired or effective speed. As another non-limiting example, the control module 110 can terminate the distribution of the acoustic energy 170 into the ROI 108, if the direction of the rotation 102 of the rolling member 115 is changed.

Various sensing and monitoring components may also be implemented within control module 110. For example, monitoring, sensing, and interface control components may be capable of operating with the motion detection system implemented within the transducer module 150, to receive and process information such as acoustic or other spatial and temporal information from the ROI 108. Sensing and monitoring components may also include various controls, interfacing, and switches and/or power detectors. Such sensing and monitoring components may facilitate open-loop and/or closed-loop feedback systems within the treatment device 100.

In some configurations, sensing and monitoring components may further include a sensor that may be connected to an audio or visual alarm system to signal overuse or impending overuse of the system. In this configuration, the system may be capable of determining the amount of energy transferred to the skin, and/or the time that the treatment device 100 has been actively emitting the energy 170. When a certain time or energy or temperature threshold has been reached, the alarm may sound an audible alarm, or cause a visual indicator to activate to alert the user that a threshold has been reached. This may control against overuse of the treatment device 100. In some configurations, the sensor may be operatively connected to the control module 110 and force the control module 110 to stop emitting the therapeutic ultrasound energy 170 from the transducer module 150.

To help facilitate movement of treatment device 100, the one or more rolling members 124 can have an outer surface, which has a coefficient of friction greater than zero. In other words, the rolling members 124 can have an outer surface, which provides grip in the target surface 106. In some examples, the rolling members 124 can be made of a rubber or silicon material or the like, and/or combinations thereof. In some configurations, the treatment device 100 can include at least two rolling members 124.

Aspects including at least two rolling members 115 can include an offset 180, which is the difference between the outer surface 151 of the transducer module wall and the target surface 106. The offset 180 can be calibrated to hold a desired thickness of coupling medium 107 between the transducer module 150 and the target surface 106.

The offset 180 can be applicable to a configuration of the treatment device 100 containing only one rolling member 115. The offset 180 can be at least 1 mm, which is measured from the target surface 106 to the outer surface 152 of the transducer module wall 151. The offset 180 can be calibrated to hold a desired thickness of coupling medium 107 between the transducer module 150 and the target surface 106.

In certain aspects, the coupling medium 107 can be water-based, light oil(s)-based, based on an emulsion of light oil and water, other coupling liquids or gels, or a combination thereof. In some applications, the coupling medium 107 can include water, light oils, or emulsions of light oil and water, which are configured to not interfere with a position sensor 140, optionally an optical position sensor 140, contained in the treatment device 100.

In certain configurations, the treatment device 100 can include a temperature sensor 142, as illustrated in Fig. 4. The temperature sensor 142 can be configured to sense the temperature of certain parts of the treatment device 100, such as the energy source 160 or the transducer module wall 151, a temperature of the target surface 106, a temperature of the ROI 108, or a combination thereof. The temperature sensor 142 can be located within the transducer module 150 or elsewhere in the treatment device 100.

In certain configurations, the treatment device 100 can include a contact sensor 144, as illustrated in Fig. 4, for sensing coupling between the transducer module 150 or the energy source 160 and the ROI 108 or the target surface 106. The contact sensor 144 can be located within the transducer module 150 or elsewhere in the treatment device 100.

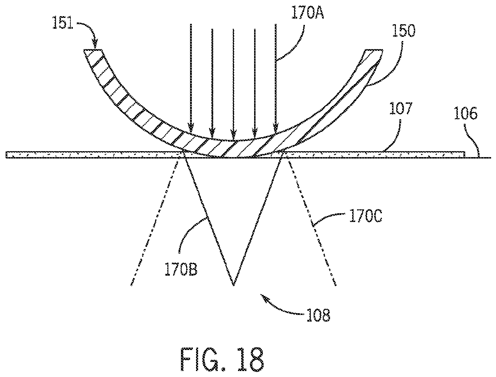

Fig. 18 illustrates a lensing effect, in accordance with some configurations. The energy 170 can be focused by the transducer module wall 151. For illustration purposes, the energy 170 is collimated and is designated by energy 170A. The collimated energy 170A can be focused by a curvature of the transducer module wall 151. Accordingly, the system 100 can produce collimated energy 170A and then can focus the original energy emission thus generating energy 170B. In another example, energy 170A can be defocused by curvature of the transducer module wall 151. Accordingly, the system 100 can produce collimated energy 170A and then can defocus the original energy emission thus generating energy 170C.

Alternatively, the energy 170 can be focused by use of a phased array in the energy source 160. The energy 170 can be focused using a lens 164 in the energy source 160, as described herein. The energy 170 can be focused by a combination of a lens 164 and a phased array in the energy source 160. The energy 170 can be collimated, focused, weakly focused, unfocused, or defocused by any combination of the energy source 160, one or more lenses 164, and the transducer module wall 151.

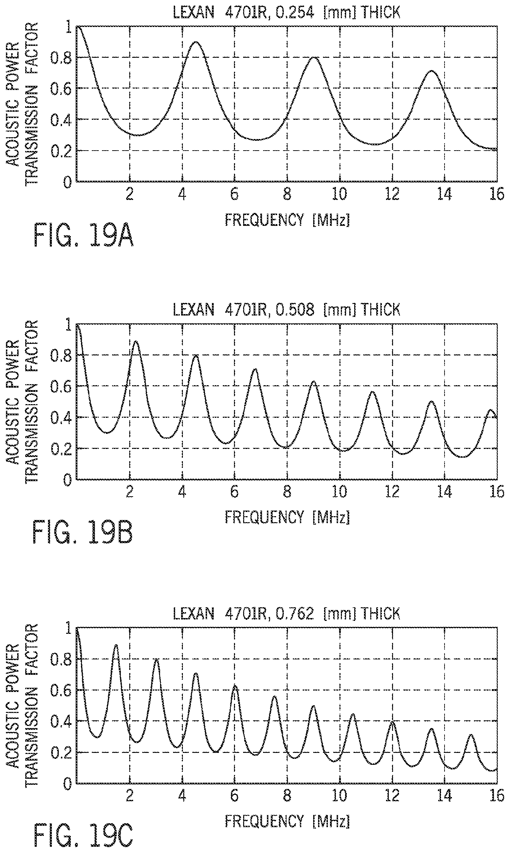

A variety of focusing, defocusing, weakly focusing, and unfocused energy can be used to generate a desired energy field directed to the ROI 108. A combination of geometric parameters of system 100 is considered to define the desired energy field directed to the ROI 108. For example, the focusing (or defocusing or unfocused) energy 170 can depend based on: on acoustic velocity of material chosen to fill the internal volume 156, the size of beam of energy 170A emitted by energy source 160, path length between energy source 160 and inner surface 155, a lens coupled to energy source 160, and a phased array as the energy source 160. In addition, the focusing (or defocusing) of energy 170B (or 170C) can depend based on acoustic velocity of material chosen for the transducer module wall 151, thickness 154 of the transducer module wall 151, and curvature (radii) of the transducer module wall 151. The frequency of energy 170A emitted by energy source 160 should be within a narrow band width for the geometry to work and produce the desired energy (170B or 170C) directed to ROI 108. Precision of the calculated geometry can be used to achieve the desired energy (170B or 170C). If the frequency of energy 170A is outside of the narrow band width, the energy 170A reflected back to energy source 160. For example, as illustrated in Figs. 19A through 19C, graphs are provided that show acoustic power transmission factor versus frequency plots for three different thicknesses. As demonstrated, the thickness of the outer wall will affect the bandwidth of the energy directed to the ROI. Lens geometry is based on the arc of the curvature of the outer wall. It should be appreciated that Lexan is not necessarily used as an acoustic window, and is used in Figs. 19A through 19C merely as an example of transmission through a window that has a thickness of approximately a simple multiple of a half wavelength.

The treatment device 100 can include wiring that is configured to provide power from the control module 110 to the energy source 160. The wiring can be configured to control the energy source 160 with the control module 110. In some configurations, a power supply can be configured to power the energy source and can be located in the transducer module 150. For example, a battery (disposable or rechargeable) can be coupled to the energy source 160 and can be configured to power the energy source 160. In one configuration, the axle 124 can include a magnet and a wire coil around the magnet, which is configured to generate electricity and is coupled to a rechargeable battery.

In certain aspects, the rolling member 115 can be a wheel, a ball or a bearing, a cylinder, a tread, or any other shape suitable for rolling along a target surface 106. In aspects incorporating a ball or a bearing for all of the rolling members 115, a treatment device 100 can be moved in any direction along the treatment surface 106.

In some configurations, the axles 124 can be moveable. The axles 124 can be configured to be sensitive to mechanical pressure on any of the transducer modules 150. At least one of the axles 124 can move in response to an increase in mechanical pressure on the treatment device 100. The axles 124 can be biased, for example, spring-loaded, to move up and down to compensate for changes in the target surface 106 above the ROI 108. In some configurations, the axles 124 can move along a z-axis in relation to the target surface 106.

In various configurations, the transducer module 150 can be configured to have an internal volume 156 that is bordered or bounded by the inner surface 155 of the transducer module wall 151. The internal volume 156 can be filled with a coupling medium 107. The transducer module 150 can further include a transducer module wall 151 including a material having a thickness 154 which may be transparent or substantially transparent to ultrasound energy. In some configurations, the transducer module 150 can include a transducer 162. In some configurations, the transducer module 150 may include an acoustic matching layer. The transducer module 150 can include a path length, which is a distance from the transducer 162 to the inner surface 155. The transducer module 150 has a thickness 154, which is a distance measured perpendicularly from the outer surface 152 to the inner surface 155.

In some configurations, the energy 170 may be delivered as a continuous wave emission. The transducer module 150 can couple the transducer 162 to ROI 108 and facilitates the transfer of the energy 170 from transducer 162 through the coupling medium 107 that is present and the transducer module wall 151 and into ROI 108. However, if the transducer module 150 is uncoupled from ROI 108 and is coupled to air, the transducer module wall can become a reflector and reflect all or at least a majority of the energy 170 back to transducer 162 as reflected energy.

The transducer module 150 can include a transducer module wall 151 having an outer surface 152 and an inner surface 155. The transducer module wall 151 can have a thickness 154 which is distance measured perpendicularly from the outer surface 152 to the inner surface 155. In various configurations, the thickness 154 can be configured to provide various physical attributes to ultrasound energy 170, as further discussed herein. The transducer module 250 can be configured to have an internal volume 156 which is bordered or bounded by the inner surface 155. The internal volume 156 can be filled with a coupling medium 107, as discussed herein.

The transducer module 150 can include an energy source 160, such as an ultrasound transducer, configured to transmit acoustic energy 170 into a region of interest 108 at and/or below a surface 106. The transducer module 150 can include an arced outer annular wall 151 having a thickness 154 of about one-half wavelength of the acoustic energy 170 and configured to encase the energy source 160 and a coupling solution 156. The transducer module 150 can include a coupling sensing system in communication with the control module 110 and configured with a prescribed distance between the source 160 and an inner surface of the transducer module 150 to determine whether the energy source 160 is coupled to the ROI 108 using an interaction of the acoustic energy 170 with the thickness 154 of about one-half wavelength. The coupling sensing system can be configured to determine whether the energy source 160 is coupled to the ROI 108 by monitoring an amount of reflection of the acoustic energy 170 back to the transducer 162 by the outer surface 151 having the thickness 154 of one-half wavelength of the acoustic energy.

In some aspects, a bottom portion of the transducer module 150 can be configured with a curved outer surface 151 which has the thickness 154 of about one-half wavelength, which is an acoustic window. In such aspects, the remainder of the transducer module 150, with the acoustic window subtracted, can be made of a material that does not or only partially transmits acoustic energy and/or has a thickness 154 greater than one-half wavelength.

In certain configurations, the treatment device 100 can be configured as a hand-held device. In one configuration, a treatment device 100 can include a transducer module 150, a position sensor 140, a tissue contact sensor, a control module 110, and a communication interface linking the transducer module 150, the sensors, and the control module 110. In one configuration, a treatment device 100 can include a position sensor 140, a control module 110, a rechargeable power supply, and a transducer module 150, which include at least one ultrasound transducer 160. In accordance with one configuration, a treatment device 100 can include a position sensor 140, a control module 110, an acoustic coupling medium dispenser 105, and a transducer module 150, which includes at least one ultrasound transducer 160. In certain configurations, the target surface 106 can be a skin surface, a subcutaneous surface, a mucosal surface, an internal organ surface, or a combination thereof.

In some configurations, the control module 110 can be capable of coordination and control of the treatment process to achieve the desired therapeutic effect on ROI 108. For example, in some configurations, the control module 110 may include power source components, sensing and monitoring components, one or more RF driver circuits, cooling and coupling controls, and/or processing and control logic components.

The control module 110 may be configured in a variety of ways to implement treatment device 100 for controlled targeting of a portion of ROI 108. For example, for power sourcing components, the control module 110 may form one or more direct current (DC) power supplies capable of providing electrical energy for the entire control module 110, including power required by a transducer electronic amplifier/driver. A DC current sense or voltage sense device may also be provided to confirm the level of power entering amplifiers/drivers for safety and monitoring purposes.

In some configurations, amplifiers/drivers may include multi-channel or single channel power amplifiers and/or drivers. In some configurations for transducer array configurations, amplifiers/drivers may also be configured with a beam former to facilitate array focusing. An exemplary beam former may be electrically excited by an oscillator/digitally controlled waveform synthesizer with related switching logic.

Power sourcing components may also include various filtering configurations. For example, switchable harmonic filters and/or matching may be used at the output of amplifier/driver to increase the drive efficiency and effectiveness. Power detection components may also be included to confirm appropriate operation and calibration. For example, electric power and other energy detection components may be used to monitor the amount of power entering transducer module 150.

Additionally, an exemplary control module 110 may further include a system processor and various digital control logic, such as one or more of microcontrollers, microprocessors, field-programmable gate arrays, computer boards, and associated components, including firmware and control software, which may be capable of interfacing with user controls and interfacing circuits as well as input/output circuits and systems for communications, displays, interfacing, storage, documentation, and other useful functions. System software may be capable of controlling all initialization, timing, level setting, monitoring, safety settings, and other system functions desired to accomplish user-defined treatment objectives. Further, various control switches, touch panels, multi-touch panels, capacitive and inductive switches, may also be suitably configured to control operation.

The control module 110 can be configured to communicate with a wireless device via wireless interface. Typically, the wireless device has a display and a user interface such as, for example, a touch screen or keyboard. Examples of a wireless or mobile device can include, but are not limited to, cell phones, a smart phones, computers, laptops, netbooks, tablets, or any other such device now known or developed in the future. Accordingly, the treatment device 100 can include any hardware, such as, for example, electronics, antenna, and the like, as well as, any software that may be used to communicate via wireless interface.

In certain configurations, the control module 110 can be a portable device. For example, the control module 110 can include a cell phones, a smart phones, computers, laptops, netbooks, tablets, or any other such device now known or developed in the future. The functions of the control module 110 can be programmed in the portable device. For example, the control module 110 functions can be downloaded as an app on the portable device and employed when using the treatment device 100. In some examples, the portable device can be interfaced to a wireless network and the control module 110 functions can be sent to the portable device. In some examples, the portable device can be interfaced to a wireless network and the portable device can be monitor over the wireless network. In some examples, the portable device can be interfaced to a wireless network and the portable device can upload data from treatments via the network.

The wireless or portable device can be configured to display an image generated by the treatment device 100. The wireless or portable device can be configured to control at least a portion of the treatment device 100. The wireless or portable device can be configured to store data generated by the treatment device 100 and sent to the wireless device.

In some configurations, the transducer module 150 can include an ultrasound transducer 162, a position sensor 140, a tissue contact sensor, a communication interface, the control module 110, a rechargeable power supply, an acoustic coupling medium dispenser 105, or any combination thereof. In certain configurations, the transducer module 150 can include at least two ultrasound transducers 162.

In certain configurations, the transducer module wall 151 can have material properties and a geometric shape to provide an ultrasound transmissive window having a thickness 154 that is a multiple of a half wavelength of the energy 170. In some configurations, the transducer module wall 151 can have a thickness 154 that is a half wavelength ("λ") of the energy 170 thick or is about a half wavelength of the energy 170 thick. In some configurations, the transducer module wall 151 can have a thickness 154 that is a multiple of a half wave length thick, such as, for example. multiples of 0, 1, 2, 3,...n of the half-wavelength. In certain configurations, the energy 170 can be an ultrasound energy, an photon-based energy, a radio-frequency energy, a microwave energy, or a combination thereof.

In some configurations, the energy source 160 can be configured with the ability to controllably produce conformal distribution of elevated temperature in soft tissue within the ROI 108 through precise spatial and temporal control of acoustic energy deposition. To this end, the control of energy source 160 may be configured within selected time and space parameters, with such control being independent of the tissue. The ultrasound energy 170 can be controlled to produce a conformal distribution of elevated temperature in soft tissue within ROI 108 using spatial parameters. The ultrasound energy 170 can be controlled to produce conformal distribution of elevated temperature in soft tissue within ROI 108 using temporal parameters. The ultrasound energy 170 can be controlled to produce a conformal distribution of elevated temperature in soft tissue within ROI 108 using a combination of spatial parameters and temporal parameters. In some configurations, a conformal distribution of elevated temperature in soft tissue within ROI 108 is conformal region of elevated temperature in ROI 108.

In various configurations, the energy source 160 can be configured to create an intensity gain from the energy source 160 to the ROI 108 of at least 100. Further, the energy source 160 can be configured to create an intensity gain from the target surface 106 to the ROI 108 of at least 5. In some configurations, the energy source 160 can be configured to focus the acoustic energy 170 into the ROI 108. As focused, the intensity from the energy source 160 to the ROI 108 can be in a range from 500 W/cm² to 25,000 W/cm². In some configurations, the energy source 160 can be configured to weakly focus the acoustic energy 170 into the ROI 108. As weakly focused, the peak intensity from the energy source 160 to the ROI 108 can be in a range from 5 W/cm² to 100 W/cm². The energy source 160 can be configured to focus the energy 170 into the ROI 108 to create an average intensity of at least 1000 W/cm² in the ROI 108. The energy source 160 can be configured to focus the energy 170 into the ROI 108 to create an intensity of at least 3 W/cm² at the target surface 106. The energy source 160 can be configured to focus the energy 170 into the ROI 108 to create an intensity of at least 10 W/cm² at the target surface 106.

In some configurations, the energy source 160 can be configured to direct the acoustic energy 170, which is defocused into the ROI 108. The energy source 160 can be configured to direct the acoustic energy 170, which is unfocused into the ROI 108. In some configurations, the energy source 160 can be configured to direct the acoustic energy 170 having a planar focus into the ROI 108.

The energy 170 can be emitted in a frequency in a range from 1 MHz to 30 MHz and for a time in a range from 1 nanosecond to 10 microseconds. Some configurations provide systems configured to direct the energy 170 into the ROI 108, which is emitted in a frequency in a range from 1 MHz to 2 GHz and for a time in a range from 100 picoseconds to 10 seconds. Some examples of these configurations provide systems configured to provide a power signal in a range of 1 kilowatt to 12 kilowatts to the energy source 160 to generate an amount of energy 170 in a range from 1 nanosecond to about 500 microseconds and in a range from about 0.5 milli-Joules to about 6 Joules, which is directed to the ROI 108. In some applications, the frequency range can be from 1 MHz to 30 MHz or can be from 1 MHz to 20 MHz or can be from 2 MHz to 10 MHz.

In many treatment applications, the frequency of the ultrasound energy 170 can be in a range from about 1 MHz to about 12 MHz, or from about 5 MHz to about 15 MHz, or from about 2 MHz to about 12 MHz or from about 3 MHz to about 7 MHz. In some applications, the frequency range can be from 1 MHz to 10 MHz or can be from 1 MHz to 7 MHz or can be from 2 MHz to 5 MHz.

The acoustic energy 170 can be emitted in an at least one increment in a range from 0.001 seconds to 5 seconds from the energy source 160 coupled to the target surface 106, wherein the acoustic energy 170 is emitted at a frequency in a range of 1 MHz to 20 MHz, at a peak intensity in a range of 5 W/cm2 to 70,000 W/cm2. The increment can be in a range from 0.001 seconds to 5 seconds and can be emitted repeatedly to deliver an amount of acoustic energy necessary to provide an effect in the tissue. The increment can be in a range from 0.001 seconds to 5 seconds and can have a delay of 10 microseconds to 1 second between each increment. The time length of time of an emission of the energy 170 into the ROI 108 for the domain of a thermal effect is in a range from milliseconds to minutes in a frequency range, as described above.

In some applications, the ultrasound energy 170 can create a thermal effect in the ROI 108. Since temperature in the ROI 108 is proportional to intensity of acoustic energy 170 that is delivered, providing a controlled delivery of the acoustic energy 170 into the ROI 108, which exceeds a thermal capacity of the medium (for example tissue) in the ROI 108, will damage or destroy the medium in the ROI 108. For example, a lesion can be created in subcutaneous tissue when the delivered ultrasound energy 170 exceeds the thermal capacity of the tissue. However, providing a controlled delivery of the acoustic energy 170 into the ROI 108, which increases the temperature in the ROI 108 of the medium but does not exceed a thermal capacity of the tissue can initiate a change of the tissue in the ROI 108 but does not destroy the tissue in the target zone of the ROI 108. For example, subcutaneous tissue can be heated to about 43 °C to about 50 °C, which can cause the collagen to shrink but does not damage or destroy the tissue in the target zone.

In some configurations, the energy 170 can create a mechanical effect in the ROI 108. A mechanical effect is a non-thermal effect in a medium created by interaction with acoustic energy 170. A mechanical effect can be one of, for example, acoustic resonance, acousto-elastic effect, acousto-mechanical effect, acoustic streaming, disruptive acoustic pressure, shock waves, histrophy, inertial cavitation, and non-inertial cavitation.

At high enough acoustic intensities, cavitation is the formation of microbubbles in a liquid portion of the ROI 108. The interaction of ultrasound field with the microbubbles can cause the microbubbles to oscillate in the ROI 108 (non-inertial (dynamic) cavitation) or to grow and eventually implode (inertial cavitation). During inertial cavitation, very high temperatures inside the bubbles occur, and the collapse is associated with a shock wave that can mechanically damage the medium (such as tissue) in the ROI 108. The time length of time of an emission of the energy 170 for the domain of cavitation is in a range from microseconds to seconds in a frequency range, as described above. In some configurations, an overlap exists, in which both of the effects, the thermal effect and the cavitation effect can occur.

The acousto-mechanical effect can cause a micro-explosion in the target zone of the ROI 108. The acousto-mechanical effect can cause an increase in a pressure in the target zone above a threshold of fragmentation of the medium in the ROI 108. A fragmentation pressure is a minimum pressure at which a substance (for example a solid) in the ROI 108 of a particular medium will explode (shatter, fragment). The time length of time of an emission of the energy 170 for the domain of an acousto-mechanical effect is in a range from nanoseconds to microseconds in a frequency range, as described above. In some configurations, an overlap exists, in which both of the effects, the cavitation and the acousto-mechanical effect can occur.

The acousto-elastic effect is an effect in a medium that arises from the combination of the pressure oscillations of an acoustic wave with the accompanying adiabatic temperature oscillations in a target zone produced by the acoustic wave. Temperature of the surrounding medium in the ROI 108 is unchanged. The acousto-elastic effect is an effect in that can overcome threshold of elasticity of the molecules in the target zone of the ROI 108. The acousto-elastic effect increases the temperature from the inside out by thermal diffusion, which can dramatically increase temperature in a target zone thus resulting in a raise in pressure in the target zone.

The acousto-elastic effect can break the thermal elastic connection of the molecules in the target zone, which can cause a micro-explosion in the target zone of the ROI 108. The acousto-elastic effect can cause raise a temperature in the target zone above a fragmentation temperature of the medium in the target zone. A fragmentation temperature is a minimum temperature at which a substance (for example a solid) in the ROI 108 of a particular medium will explode (shatter, fragment). The time length of time of an emission of the energy 170 for the domain of an acousto-mechanical effect is in a range from picoseconds to microseconds in a frequency range, as described above. In some configurations, an overlap exists, in which both of the effects, the acousto-mechanical effect and the acousto-elastic effect can occur.

In some configurations, energy 170 can be ultrasound energy. In some configurations, the energy 170 can be more than one energy, for example, but not limited to ultrasound energy and a photon-based energy. Of course the energy 170 is not limited to only one energy or to two different energy types, for example, the energy 170 can include a number of energy types and/or can include a particular energy type at different frequencies. The energy source 160 can be configured to emit ultrasound energy. The energy source 160 can be configured to emit ultrasound energy and to emit a photon-based energy.

In some configurations, energy source 160 can be configured to emit at least two different forms of energy 170. For example, the energy source 160 can be configured to emit ultrasound energy into a portion of a treatment region and the energy source 160 can be configured to emit a second energy, which is different than ultrasound energy, into a portion of the treatment region. For example, the second energy can be provided by a laser, or an intense pulsed light (IPL), or a light emitting diode (LED), or a radio frequency source, or a microwave energy source, or a plasma source, or a magnetic resonance source, or a mechanical energy source, or any other photon-based energy source. In some applications, the energy source is a cryogenic source to provide treatment by cooling the targeted tissue. The second energy can be provided by any appropriate energy source now known or created in the future. The energy source 160 can be configured to emit a third energy, which can be provided by any source described herein. The energy source 160 can be configured to emit an nth energy, which can be provided by any source described herein.

In some configurations, the energy source 160 can include an ultrasound energy source and a photon-based energy source. For example, the photon-based energy source can be a laser. In some examples, the photon-based energy source can be IPL. In some examples, the photon-based energy source can be one or more LEDs. In some examples, the photon-based energy source can be a nanosecond Q-switch laser. In some examples, the photon-based energy source can be a picosecond Q-switch laser.

The photon-based energy source can be configured to emit "blue light" having a wavelength from about 400 nanometers to about 440 nanometers. Blue light may be applied as a pretreatment before the ultrasound energy is applied. Blue light may be applied concurrently with the ultrasound energy. The photon-based energy source can be configured to emit "red light" having a wavelength from about 600 nanometers to about 1350 nanometers. Red light may be applied as a pretreatment before the ultrasound energy is applied. Red light may be applied concurrently with the ultrasound energy. The photon-based energy source can be configured to emit ultraviolet (UV) energy having a wavelength from about 100 nanometers to about 400 nanometers. UV light may be applied as a pretreatment before the ultrasound energy is applied. UV light may be applied concurrently with the ultrasound energy.

In some configurations, the energy source 160 can include an ultrasound energy source and a RF energy source. In some configurations, the energy source 160 can include an ultrasound energy source and a microwave energy source. In some configurations, the energy source 160 can include an ultrasound energy source and a plasma source. In some configurations, the energy source 160 can include an ultrasound energy source and a magnetic resonance source energy source. In some configurations, the energy source 160 can include an ultrasound energy source and a mechanical energy source.

In some configurations, ultrasound energy 170 is a continuous wave emission. The transducer module 150 can couple the transducer 162 to ROI 108 and facilitates the transfer of the energy 170 from transducer 162 through any coupling medium 107 that is present and the transducer module wall 151 and into ROI 108. However, if the transducer module 150 is uncoupled from ROI 108 and is coupled to air, the transducer module wall can become a reflector and reflect all or at least a majority of the energy 170 back to transducer 162 as reflected energy.

In some configurations, a digital synthesizer can be coupled to transducer 162 and configured to frequency sweep transducer 162. In some configurations, frequency sweep can monitor the constant average output power of transducer 162. In some configurations, frequency sweep can be a step function of a set of different frequencies. In some configurations, the frequency sweep is a chirp function. In some configurations, the step function of a set of different frequencies can include a plurality of different frequencies.

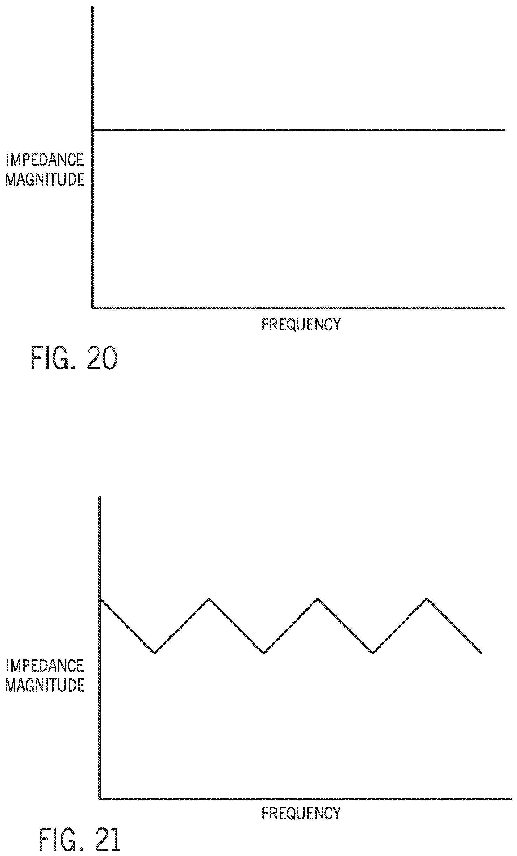

In some configurations, an output from the frequency sweep can be monitored, as illustrated in Figs. 20 and 21. The units for axis of the graphs in Figs. 20 and 21 are electrical impedance in the y axis and frequency in the x axis. Fig. 20 illustrates the feedback from a frequency sweep when transducer module 150 is coupled to ROI 108. Fig. 21 illustrates the feedback from a frequency sweep when transducer module 150 is uncoupled (or coupled to air). This difference in feedback from the frequency sweep can be a coupling detection system. If the frequency sweep reports feedback is similar to Fig. 20 device 100 continues to function providing ultrasound energy 170 to ROI 108. If the frequency sweep reports feedback is similar to Fig. 21, the transducer 162 is shut down as a safety mechanism and/or to protect the transducer 162 from being damaged or destroyed.

In some configurations, the system can monitor and adjust output power to achieve a constant average output power, even with variations in the medium temperature and/or transducer temperature. In some configurations, the transducer module 150 can include a temperature sensor 142. In some configurations, transducer module 150 can include two temperature sensors 142. For example, one of the temperature sensors 142 can be in contact with coupling medium 107 and the second temperature sensor 142 can be in contact with transducer 162. In some configurations, if the temperature as reported by the temperature sensor is above, for example, 43 °C, the transducer 162 stops emission of ultrasound energy 170.

In certain configurations, the offset 180 can be used with the acoustic coupling medium dispenser 105, as discussed herein, to ensure the proper thickness of the coupling medium 107 is applied to the target surface 106. In some configurations, the acoustic coupling medium dispenser 105 can be integrated into traducer module 150. However, the acoustic coupling medium dispenser 105 can be placed in a variety of positions in system 100, as long dispenser 105 can provide coupling medium 107 to at least one of the transducer module 150 and the surface 106. The offset 180 can be used with a contact sensor, such as the contact sensors described herein, to provide coupling of the transducer 162 to the ROI 108 during use of the system 100.

In certain configurations, the position sensor 140 may be located behind a transducer module 150, in front of a transducer module 150, or integrated into a transducer module 150. The ultrasound transducer module 150 may include more than one position sensor 140, such as, for example, a laser position sensor and a motion sensor, or a laser position sensor and a visual device, or a motion sensor and a visual device, or a laser position sensor, a motion sensor, and a visual device.

In some configurations, the position sensor 140 can include a visual element such as a camera or video capture device. In some configurations, the position sensor 140 can include a laser position sensor. In some configurations, the position sensor 140 can include a Doppler laser position sensor. In some configurations, the position sensor 140 can include a 3D magnetic sensor. For example, an optical position sensor can track position like a computer mouse that uses a laser sensor as opposed to an older version of a mouse with a roller ball. The position sensor can communicate position data versus time to a display to track a position of ultrasound transducer module 150, such as, for example, overlaid on an image of ROI 108, overlaid on an image of target surface 106, as referenced to tagged or pre-identified features, as reference to injury location, as referenced to a prior treatment, and combinations thereof. A treatment plan includes a movement pattern of ultrasound transducer module 150. Such a movement pattern can be displayed and the position sensor tracks a position of ultrasound transducer module 150 during treatment as compared to the movement pattern. Tracking the ultrasound transducer module 150 with position sensor and comparing the tracked movement to a predetermined movement may be useful as a training tool. In some configurations, laser position sensor can tag or identify a feature on target surface 106.

In some configurations, the position sensor 140 may determine a distance between pulses of therapeutic ultrasound energy 170 to create a plurality of treatment zones that are evenly spaced or disposed in any spatial configuration in 1-D or 2-D patterns. As the ultrasound transducer module 150 is moved in a direction 104, the position sensor 140 determines distance, regardless of a speed that ultrasound transducer module 150 is moved, at which a pulse of therapeutic ultrasound energy 170 is to be emitted in to ROI 108.

In some configurations, two or more position sensors 140 can be used to determine the movement speed of two or more rolling members 115. In such configurations, the controller can be configured to alter or terminate the distribution of energy 170 into the region of interest 108 if the speed of a first rolling member 115 is substantially different than a speed of a second rolling member 115, if a speed of either or both of the two or more rolling members 115 is below a minimum speed, if a speed of either or both of the two or more rolling members is above a maximum speed, if a direction of movement of a first rolling member 115 is different than a direction of movement of a second rolling member 115, or any combination thereof.

In certain aspects, a remote position sensor, a remote orientation sensor, or a remote position and orientation sensor can be deployed to provide position and/or orientation information regarding the treatment device 100. For example, a camera can acquire images from two or more angles, and the acquired images can be processed to determine a position and orientation of the treatment device 100.

In certain aspects, the control module 110 can store past treatment positions. The control module 110 can use stored treatment positions to avoid retreating previously treated positions, if necessary.

In some configurations, the transducer module 150 can include a contact sensor 144. In some configurations, the contact sensor 144 can communicate whether the transducer module 150 is coupled to the ROI 108. The contact sensor 144 may measure a capacitance of a target surface 106 above the ROI 108 and communicate a difference between the capacitance of the contact to the target surface 106 and the capacitance of air. In some configurations, the tissue contact sensor can be initiated or turned on by pressing the ultrasound transducer module 150 against the target surface 106.

Various configurations provide methods of sensing coupling of an energy source 160 to a ROI 108. For example, a method of sensing coupling includes providing the energy source 160 having a transducer 162, providing the transducer module 150 having a substantially curved outer surface with a thickness 154 of about one-half wavelength of the acoustic energy and configured to encase the transducer 162 and a coupling medium, and performing a frequency sweep function. The method can include emitting ultrasound energy 170 from the transducer 162; receiving reflected energy 170; frequency sweeping the transducer; determining the feedback from the frequency sweep is below (or above depending where the level is set) a threshold level; and determining if the transducer 162 is coupled to the ROI 108.

In some configurations of the method, if the feedback from the frequency is above the threshold level, then the transducer 162 is not coupled to the ROI 108. In some configurations, if the feedback from the frequency is above (or below depending where the level is set) the threshold level, then the transducer 162 is coupled to the ROI 108. The method can further include providing constant average output power from the transducer 162. The method can further include terminating power to the transducer 162. The sweep frequency has a period, which is calculated using a path length of the standoff and the speed of sound.

Various configurations provide a contact sensor 144 for determining whether an ultrasound source 160 is coupled to a target 108. In some configurations, the coupling sensing system includes an ultrasound source 160 having a transducer 162, an acoustically transparent standoff (transducer module 150) coupled to the transducer 162 including a substantially curved outer surface having a thickness 154 of about one-half wavelength of the acoustic energy at a bottom surface of the standoff. The substantially curved outer surface having a thickness 154 of about one-half wavelength is a reflector when the ultrasound source 162 is not coupled to the ROI 108. The substantially curved outer surface having a thickness 154 of about one-half wavelength is transparent to ultrasound energy 170 when the ultrasound source 162 is coupled to the ROI 108.

In various configurations, the contact sensor 144 can be combined with one or more different sensing techniques. For example, the contact sensor 144 can be combined with a hall detector. For example, the contact sensor 144 can be combined with an optical detector. For example, the contact sensor 144 can be combined with a conductive detector. For example, the contact sensor 144 can be combined with a piezo electric detector. For example, the contact sensor 144 can be combined with a mechanical detector. For example, the contact sensor 144 can be combined with a magnetic detector. In various configurations, the coupling detection system can be combined with at least one of a hall detector, optical detector, an acoustic impedance detector, a conductive detector, a piezo electric detector, a mechanical detector, a magnetic detector, an acoustic impedance detector, and combinations thereof.

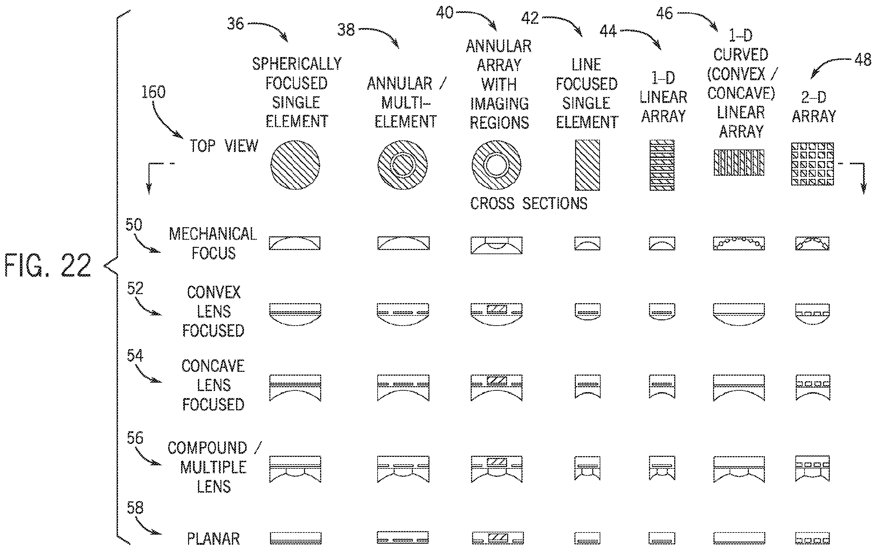

Referring to Fig. 22, a plurality of exemplary energy source 160 configurations is illustrated in accordance with various configurations. In some configurations, the energy source 160 includes at least one ultrasound transducer 162. In some configurations, energy source 160 includes at least one ultrasound transducer and a focusing device 164, such as, for example, a lens.

For example, a transducer 162 may include a spherically focused single element 36. The transducer 162 may include an annular/multi-element array 38. The transducer 162 may include an annular array with an imaging region 40. A transducer 162 may include a line-focused single element array 42. The transducer 162 may include a 1-D linear array 44. The transducer 162 may include a 1-D curved (convex or concave) linear array 46. The transducer 162 may include a 2-D array 48.

With further reference to Fig. 22, the previous described configurations of a transducer 162 can be coupled to a lens 164. For example, the lens 164 can be a mechanical focus lens 50. the lens 164 can be a convex focus lens 52. The lens 164 can be a concave focus lens 54. The lens 164 can be a compound/multiple focused lens 56. The lens 164 can be a planar lens 58. The transducer 162 may be individually or in combination coupled to a lens 164 to provide at least one of a focused ultrasound energy 170, an unfocused ultrasound energy 170, or a defocused ultrasound energy 170.

In some configurations the transducer module 150 can include at least one lens 164. For example, the energy source 160 can be configured with at least one lens 164 that is configured to focus the energy 170 within the ROI 108. The energy source 160 and the at least one lens 164 can be configured in a variety of ways depending on various target treatments that may include such parameters as, for example, a desired depth of energy 170, a type of focusing of energy 170, a number of lines of energy 170, and a size of the transducer module 150.

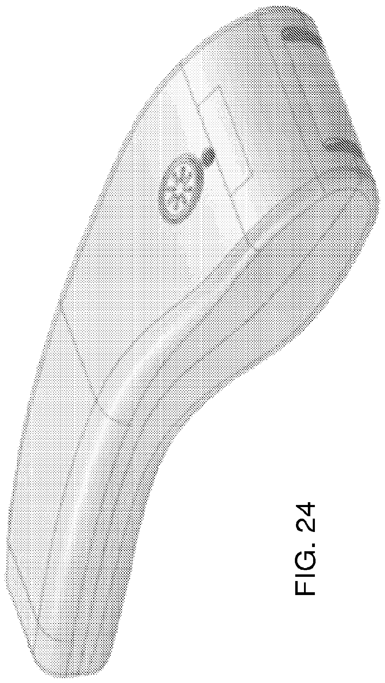

In some configurations, the lens 164 can be configured to focus the energy 170 into the ROI 108. The lens 164 can be configured to weakly focus the energy 170 into the ROI 108. The lens 164 can be configured to direct the energy 170, which is defocused, into the ROI 108. The lens 164 can be configured to direct the energy 170, which is unfocused, into the ROI 108. The lens 164 can be configured to direct the energy 170 having a planar focus into the ROI 108. Additional examples of transducer 162 and lens 164 configurations are discussed in the description of Fig. 24.

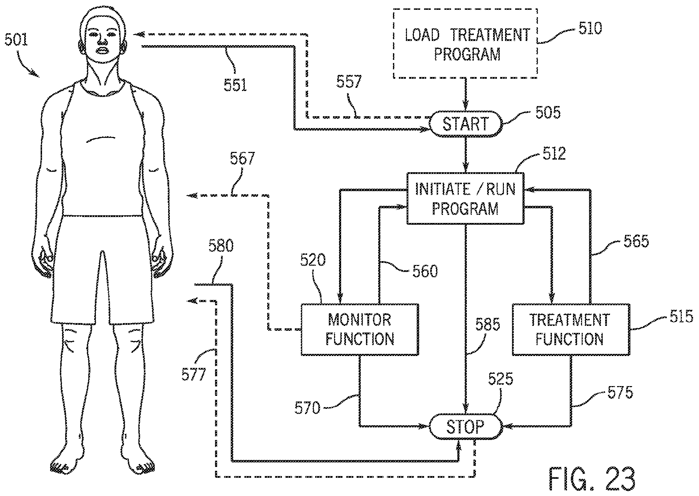

Moving to Fig. 23, a flow chart illustrates an exemplary operation of the treatment system, according to various configurations. The process includes a treatment start 505 and a treatment stop 525. A treatment program 510 can be loaded into the control module 110 to start the process 505. The treatment program 510 can be loaded during manufacture of the treatment device 100, which locks the device 100 into only the provided treatment program 510. However, the treatment program 510 may be selected by user 501. The treatment program 510 instructs the control module 110 to provide programmed distribution of energy 170 in the ROI 108 for the desired treatment. The treatment program 510 can include parameters for the treatment function 515. The treatment program 510 can include a treatment map, whereby a user can designate a starting position on the treatment map.

To use the treatment device 100, the user 501 interfaces 551 with the treatment device 100 to provide a treatment start signal 505 that initiates and/or run the treatment program 510 and provides open-loop feedback 557 to the user 501 that treatment has started, such as, a signal or an indicator, which could be, for example, a lit LED or a message on a screen.

As the treatment device 100 runs treatment program 512, the control module 110 operates a monitor function 520, which provides closed-loop feedback 560 with the treatment program 512. If feedback 560 is provided to indicate a positive signal, the run treatment program 512 continues. If the feedback 560 provides a negative signal, a stop signal 525 is sent to the control module 110 and the treatment program 512 is terminated. To this end, the system may provide closed-loop feedback and control. The monitor function 520 can also provide an open-loop feedback 567 to the user 501, which may include a system ready indication, a system operating within programmed parameters indication, and/or a system fault indication. For example, such feedback 567 may be communicated by, for example, LEDs and/or in text or symbol format displayed on a screen of the treatment device 100 or in communication with the treatment device 100 or mobile device, such as, for example a smart phone or a tablet device.

The monitor function 520 can monitor temperature of the transducer module 150 and relative to a threshold. The monitor function 520 can monitor battery power and provide a lower threshold. The monitor function 520 can monitor a number of lines of treatment provided by energy source 160 and provide a threshold, which is an upper limit of the number lines. The monitor function 520 can monitor a total use time and provide a time threshold. The monitor function 520 can monitor the treatment system electronics and or mechanics and provide a fault signal. The monitor function 520 can monitor any other operational parameter or basic safety parameter and provide a positive or negative signal based on the parameter status via the closed-loop feedback 560. Thus, the monitor function 520 can monitor the operation of the device 100, including treatment parameters and non-treatment parameters. The threshold or limit provides a decision point for the monitor function 520.

Various sensing and monitoring components may also be implemented within the monitoring function 520. For example, the monitoring function 520 may be capable of operating with various motion detection systems implemented within treatment device 100, to receive and process information such as acoustic or other spatial and temporal information from ROI 108. The monitoring function 520 may also include various controls, interfacing, and switches and/or power detectors.

In some configurations, the monitoring function 520 may further include a sensor that may be connected to an audio or visual alarm system to prevent overuse of system. For example, the sensor may be capable of sensing the amount of energy transferred to the skin, and/or the time that the treatment device 100 has been actively emitting energy 170. When a certain time or temperature threshold has been reached, the monitor function 520 can provide an open-loop feedback 567 to alert the user 501 that the threshold has been reached, which may include one of more of an audible alarm, a visual indicator, or an alphanumeric message. The threshold can be used to control overuse of treatment device 100. The monitoring function 520 can provide closed-loop feedback 560, such as, for example, the sensor may be operatively connected to control module 110 and force control module 110, to stop emitting ultrasound energy 170 from ultrasound transducer module 150. In some configurations that include the coupling medium application device 105, the empty sensor 277 can provide an open-loop feedback to the monitor function 520 and the control module 110.

As the treatment device 100 runs treatment program 512, the control module 110 can operate a treatment function 515, which provides closed-loop feedback 560 with the treatment program 512. If closed-loop feedback 565 provides a positive signal, the treatment program 512 continues. If the closed-loop feedback 565 provides a negative signal, a stop signal 525 is sent to the control module 110 and the treatment program 512 is terminated. The treatment function 515 can utilize the treatment parameters and thresholds, which provide closed-loop feedback, which can be either positive or negative signals based on the thresholds. If a treatment parameter of the treatment function 515 goes over the threshold or limit, a stop treatment signal 525 and the control module 110 terminates the treatment program 512.

For example, treatment function 515 can provide the control module 110 the parameters of movement of the transducer module 150. For example, parameters of movement, such as, a minimum speed, a maximum speed, and/or a change of direction, may be included in the treatment function 515. The treatment function 515 can provide spatial parameters and/or temporal parameters of the energy source 160, to provide a programmed distribution of energy 170 in the ROI 108. If the system is not operating within these parameters, a stop treatment signal 525 and the control module 110 can terminate the treatment program 512. The treatment function 515 can send a stop signal 585 at the end of the programmed treatment. The end of the programmed treatment maybe based on a time period, a total amount of energy delivered, or a total amount of area treated, or any combination thereof.

If the stop 525 has been initiated by either the monitor function 520, the treatment function 515, or the user 501, an open-loop feedback 577, such as, a stop indicator is communicated to the user 501. The user 501 can interface 580 with the treatment device 100 to send a stop signal 525 to control module 110.

In an example, the control module 110 is configured to receive at least one communication and control a distribution of the acoustic energy 170 transmitted by the transducer 162. A coupling sensing system, in communication with the control module 110, can be configured to determine whether the transducer 162 is coupled to the ROI 108 by monitoring an amount of reflection of the acoustic energy 170 back to the transducer 162 by the outer surface 152 of the module wall 151, which has a thickness 154 of about one-half wavelength. The position sensor 140, in communication with the control module 110, can be configured to monitor the speed of rotation of the transducer module 150. A treatment function 515 can be configured to send a positive signal the control module 110 via closed-loop feedback 565, if the coupling sensing system communicates the transducer 162 is coupled to the ROI 108, and if the speed of the rotation of the transducer module 150 is greater than a programmed minimum speed or is less than a programmed maximum speed.

The treatment function 515 can further be configured to send a positive signal the control module 110 via closed-loop feedback 565, if the speed of the rotation of the transducer module 150 is above a minimum speed. The treatment function 515 can further be configured to send a positive signal the control module 110 via closed-loop feedback 565, if the speed of the rotation of the transducer module 150 is below a maximum speed. The treatment function 515 can further be configured to send a positive signal the control module 110 via closed-loop feedback 565, if the direction of the rotation of the transducer module 150 is unchanged.

The treatment function 515 can further be configured to send a stop signal 525 to the control module 110, if the coupling sensing system communicates the transducer 162 is not coupled to the ROI 108. The treatment function 515 can further be configured to send a stop signal 525 to the control module 110, if the speed of the rotation of the transducer module 150 is below the minimum speed. The treatment function 515 can further be configured to send a stop signal 525 to the control module 110, if the speed of the rotation of the transducer module 150 is above the maximum speed. The treatment function 515 can further be configured to send a stop signal 525 to the control module 110, if the direction of the rotation of the transducer module 150 has changed.

In an example, the treatment program 512 can be configured to initiate the treatment stop command 525 after the transducer module 150 and the portion of the treatment device 100, as reported by the second position sensor move a programmable and defined distance. The treatment device 100 can be configured to maintain the distribution of the acoustic energy 170 into the ROI 108, if the coupling sensing system communicates the transducer 162 is coupled to the ROI 108, and if the first position sensor 140 and the second position sensor communicate the movement of the transducer module 150 is substantially similar to the movement of the portion of the treatment device 100, as reported by the second position sensor.

In an example, the treatment program 512 can be initiated by the treatment start command 505 configured to signal the control module 110 to initiate the distribution of the acoustic energy 170 into the at least one of the target surface 106 and the ROI 108. The treatment stop command 525 causes the control module 110 to terminate the distribution of the acoustic energy 170 into the at least one of the target surface 106 and the ROI 108. The treatment function 515 can be configured to initiate the treatment stop command, if the coupling detector communicates the transducer 162 is not coupled to the ROI 108, or if the speed of the transducer module 150 along the surface 106 is substantially different than the speed reported by the second position sensor, or if the position of the transducer module 150 is substantially different than the position reported by the second position sensor.

In an example, the treatment program 512 can be configured to maintain the distribution of the acoustic energy 170 into the ROI 108, if the speed of the transducer module 150 is above a minimum speed. The treatment device 100 can be configured to maintain the distribution of the acoustic energy 170 into the ROI 108, if the speed of the transducer module 150 is below a maximum speed. The treatment program 512 can be configured to maintain the distribution of the acoustic energy 170 into the ROI 108, if a direction of the rotation of the transducer module 150 is unchanged. In an example, the treatment program 512 can be configured to maintain the distribution of the acoustic energy 170 into the at least one of the target surface 106 and the ROI 108, if the coupling sensing system communicates the transducer 162 is coupled to at least one of the target surface 106 and the ROI 108, and if the speed the rotation of the transducer module 150 is below the maximum speed.

As used above, "substantially different" can be a difference of at least 10%-15%. However, at slower speeds of the wheels, substantially different can be a difference of at least 5%, However, with the use of digital position sensors and/or 128 position stepper devices, substantially different can be a difference of at least 2% or less, such as at least 1%. Substantially similar can be a difference of less than 10%-15%. However, at slower speeds of the wheels, substantially similar can be a difference of less than 5%. However, with the use of digital position sensors and/or 128 position stepper devices, substantially similar can be a difference of less than 2% or less, such as less than 1%. The actual percentage for the difference is limited by the accuracy of the position sensors, which are used in the treatment device 100. Position sensors with better accuracy allow for lower percentages for the differences. In an example of a movement of the device 100 having slow speed of 1 cm/sec, the difference of 15% (1.5 mm) may be acceptable. However, as speeds increases, the percentage of the difference needs to be lower and more accurate positions sensors are used. The accepted difference is based on the speed of the movement of the device 100 and the pitch of the treatment lines created by the energy 170.

Some configurations provide a method configured for treating a desired ROI including a target volume below large area of target surface 106. For example, the ROI can be at least a portion of the legs and buttocks during a treatment. For example, the ROI can be at least a portion of midsection during a treatment. Some configurations provide a treatment device 100 configured to treat a large surface area 106 and includes a plurality of transducer modules 150, each of which are coupled to a control module 110.

In some configurations, the method can further include initiating at least one thermal effect in the ROI 108. The at least one thermal effect is the thermal effect is one of heating in the ROI 108, or creating a conformal region of elevated temperature in the ROI 108. In some configurations, the thermal effect is one of lesion creation in the ROI 108, tissue necrosis in a portion of the ROI 108; coagulation tissue in the ROI 108, or exceeding a thermal capacity of tissue in a portion of the ROI 108, and combinations thereof.

In some configurations, the method can further include initiating at least one mechanical effect in the ROI 108. The at least one mechanical effect is at least one of cavitation, vibration, hydrodynamic, resonance-induced, streaming, vibro-accoustic stimulation, a pressure gradient and combinations thereof.

In some configurations, the method can further include providing at least one biological effect in the ROI 108 based in the temperature function. The at least one biological effect can be destroying tissue in the at least a portion of the ROI 108.

In some configurations, the method can further include monitoring of results of the acoustic tissue treatment during the delivery of acoustic energy 170. The method can further include monitoring of results of the acoustic tissue treatment after the delivery of acoustic energy 170. The method can further include planning of additional treatment.

The above-described design is particularly useful for larger body areas such as treatment of cellulite/fat in hips and buttocks or treatment of the abdomen. However, a smaller design could be useful in treatments on the face or other areas with a smaller surface or treatment area.

### Example 1.

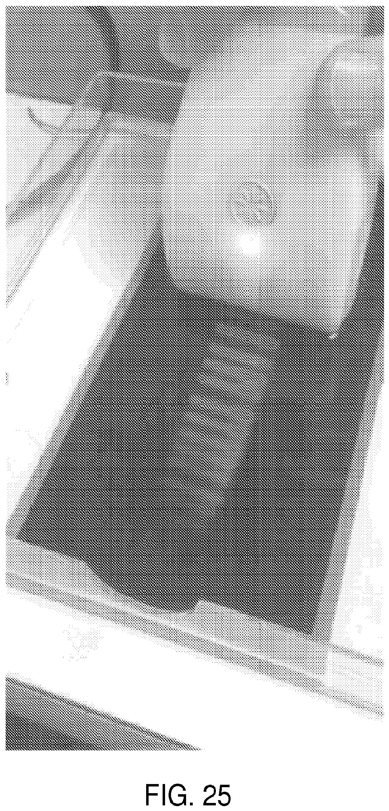

A device was prepared in accordance with the principles described above, and as shown in Fig. 24. The device contains a 5 MHz cylindrically focused ultrasound source that is 20 mm wide and 8 mm in height. The ultrasound source has a plastic lens configured to focus emitted ultrasound at a depth of 3 mm below a target surface. A pair of 12.5 mm diameter wheels at each end of the ultrasound source serve as position encoders via magnetic rotation sensors. The device includes a switch to enable treatment. The control module determines when the device has been moved a programmable distance and emits a treatment line. When the device is moved, a series of treatment lines are provides, as shown in Fig. 25 where the device is coupled to a thermally sensitive plastic. A radius of a half wavelength thick acoustic window is about 6.5 mm.

The present invention has been described above with reference to various exemplary configurations. However, those skilled in the art will recognize that changes and modifications may be made to the exemplary configurations without departing from the scope of the present invention. For example, the various operational steps, as well as the components for carrying out the operational steps, may be implemented in alternate ways depending upon the particular application or in consideration of any number of cost functions associated with the operation of the system, e.g., various of the steps may be deleted, modified, or combined with other steps. Further, it should be noted that while the method and system for ultrasound treatment as described above is suitable for use by a medical practitioner proximate the patient, the system can also be accessed remotely, i.e., the medical practitioner can view through a remote display having imaging information transmitted in various manners of communication, such as by satellite/wireless or by wired connections such as IP or digital cable networks and the like, and can direct a local practitioner as to the suitable placement for the transducer. Moreover, while the various exemplary embodiments may comprise non-invasive configurations, system can also be configured for at least some level of invasive treatment application. These and other changes or modifications are intended to be included within the scope of the present invention, as set forth in the following claims.

## Claims

1. A treatment device (100) for delivering an ultrasound energy (170) into a region of interest (108) at or beneath a target surface (106), the treatment device (100) comprising:
a housing (112);
a transducer module (150) coupled to the housing (112), the transducer module (150) comprising an ultrasound energy source (160) configured for delivery of the ultrasound energy, the delivery of the ultrasound energy being pulsed, the transducer module (150) having a transducer module wall (151) that includes a curved surface having a non-zero radius of curvature, the treatment device (100) movable relative to the target surface (106) while retaining contact between the curved surface and the target surface (106),
one or more position sensors (140) in communication with a control module (110) and configured to transmit a position signal representative of a position of the treatment device (100) relative to the target surface (106); and
the control module (110) configured to control the ultrasound energy source (160),
wherein the position sensor (140) is configured to track a position of the ultrasound transducer module (110) during treatment as compared to a movement pattern of the ultrasound transducer module which is part of a treatment plan and calculate a distance traveled along the target surface (106),
wherein the position sensor (140) or the control module (110) is configured to determine a distance between pulses of therapeutic ultrasound energy to create a plurality of treatment zones, and
wherein the position sensor (140) or control module (110) is configured to determine the distance, regardless of the speed that the transducer module (150) is moved, at which a pulse of therapeutic ultrasound energy is to be emitted.

2. The treatment device (100) according to claim 1, wherein the transducer module (150) is sealed and contains a coupling medium to facilitate coupling of the ultrasound energy source with the region of interest.

3. The treatment device (100) according to claim 1, the treatment device (100) further comprising a contact sensor (144) in communication with the control module (110) and configured to determine when the ultrasound energy source is acoustically coupled to the region of interest.

4. The treatment device (100) according to claim 3, wherein the control module (110) is configured to provide the termination signal that terminates the delivery of the ultrasound energy if the contact sensor (144) determines that the ultrasound energy source is uncoupled to the region of interest.

5. The treatment device (100) according to claim 1, wherein the control module (110) is configured to provide the termination signal that terminates the delivery of the ultrasound energy if the position of the treatment device (100) is outside a pre-defined area of treatment.

6. The treatment device (100) according to claim 1, the device (100) further comprising a rolling member (115) and an axle (124) positioned through a center axis of the rolling member (115).

7. The treatment device (100) according to claim 1, wherein the control module (110) is configured to terminate delivery of the ultrasound energy if the speed of the treatment device (100) is greater than an upper threshold.

8. The treatment device (100) according to claim 1, wherein the ultrasound energy source is configured to deliver the ultrasound energy through an acoustic window having a thickness of about one half wavelength of the ultrasound energy or about a simple multiple of one half wavelength of the ultrasound energy.

9. The treatment device (100) according to claim 8, wherein the control module is configured to provide a termination signal to terminate delivery of the ultrasound energy if it is determined that the ultrasound energy is reflected by the acoustic window when the ultrasound energy source is uncoupled from the region of interest.

10. The treatment device (100) according to claim 1, the treatment device (100) further comprising a rolling member (115) coupled to the housing (112), the treatment device (100) being movable relative to the target surface (106) by rotating the rolling member (115) along the target surface (106) in a rotational direction while retaining coupling between the ultrasound energy source and the region of interest.

11. The treatment device (100) according to claim 10, wherein the rolling member (115) is disposed outside an emission path of the ultrasound energy from the ultrasound energy source.

## Patentansprüche

1. Eine Vorrichtung zur Behandlung (100) zum Abgeben einer Ultraschallenergie (170) in einen interessierenden Bereich (108) an oder unter einer Zieloberfläche (106), wobei die Vorrichtung zur Behandlung (100) ferner umfasst:
ein Gehäuse (112);
ein Wandler Modul (150), das mit dem Gehäuse (112) gekoppelt ist, und wobei das Wandler Modul (150) eine Ultraschallenergiequelle (160) umfasst, die für die Abgabe der Ultraschallenergie konfiguriert ist, wobei die Abgabe der Ultraschallenergie gepulst ist, und wobei das Wandler Modul (150) eine Wandler-Modul-Wand (151) aufweist, die eine gekrümmte Oberfläche mit einem Krümmungsradius ungleich Null aufweist, wobei die Vorrichtung zur Behandlung (100) relativ zu der Zieloberfläche (106) beweglich ist, während der Kontakt zwischen der gekrümmten Oberfläche und der Zieloberfläche (106) beibehalten wird; und
einen oder mehrere Positionssensoren (140), die mit einem Steuerung Modul (110) in Verbindung stehen und in der Art und Weise konfiguriert sind, dass sie ein Positionssignal übertragen, das eine Position der Vorrichtung zur Behandlung (100) relativ zu der Zielfläche (106) darstellt; und
das Steuerung Modul (110) konfiguriert ist, um die Ultraschallenergiequelle (160) zu steuern, wobei der Positionssensor (140) konfiguriert ist, um eine Position des Ultraschall Wandler Moduls (110) während der Behandlung im Vergleich zu einem Bewegungsmuster des Ultraschall Wandler Moduls das ein Teil eines Behandlungsplans ist, zu verfolgen und eine entlang der Zieloberfläche (106) zurückgelegte Strecke zu berechnen; und
wobei der Positionssensor (140) oder wobei das Steuerung Modul (110) in der Art und Weise konfiguriert ist, dass er oder sie einen Abstand zwischen Impulsen therapeutischer Ultraschallenergie bestimmt, um eine Vielzahl von Behandlungszonen zu erzeugen, und wobei der Positionssensor (140) oder das Steuerung Modul (110) in der Art und Weise konfiguriert ist, dass er oder sie unabhängig von der Geschwindigkeit, mit der das Wandler Modul (150) bewegt wird, den Abstand bestimmt, mit dem ein Impuls von therapeutischer Ultraschallenergie ausgesendet werden soll.

2. Die Vorrichtung zur Behandlung (100) nach dem vorhergehenden Anspruch 1, wobei ferner das Wandler Modul (150) versiegelt ist und ferner ein Kopplungsmedium enthält, um die Kopplung der Ultraschallenergiequelle mit dem interessierenden Bereich zu erleichtern.

3. Die Vorrichtung zur Behandlung (100) nach dem vorhergehenden Anspruch 1, wobei ferner die Vorrichtung zur Behandlung (100) einen Kontaktsensor (144) umfasst, der ferner mit dem Steuerung Modul (110) in Verbindung steht und ferner in der Art und Weise konfiguriert ist, dass er dabei feststellt, wann die Ultraschallenergiequelle akustisch mit dem interessierenden Bereich gekoppelt ist.

4. Die Vorrichtung zur Behandlung (100) nach dem vorhergehenden Anspruch 3, wobei ferner das Steuerung Modul (110) in der Art und Weise konfiguriert ist, dass das Steuerung Modul (110) das Beendigungssignal bereitstellt, das dabei die Abgabe der Ultraschallenergie beendet, wenn der Kontaktsensor (144) ferner feststellt, dass die Ultraschallenergiequelle nicht an den interessierenden Bereich gekoppelt ist.

5. Die Vorrichtung zur Behandlung (100) nach dem vorhergehenden Anspruch 1, wobei ferner das Steuerung Modul (110) in der Art und Weise konfiguriert ist, dass das Steuerung Modul (110) das Beendigungssignal bereitstellt, das die Abgabe der Ultraschallenergie beendet, wenn die Position der Vorrichtung zur Behandlung (100) außerhalb eines vorher festgelegten Behandlungsbereichs liegt.

6. Die Vorrichtung zur Behandlung (100) nach dem vorhergehenden Anspruch 1, wobei ferner die Vorrichtung (100) ferner ein Rollteil (115) und eine Achse (124) umfasst, die durch eine Mittelachse des Rollteils (115) positioniert ist.

7. Die Vorrichtung zur Behandlung (100) nach dem vorhergehenden Anspruch 1, wobei ferner das Steuerung Modul (110) in der Art und Weise konfiguriert ist, dass das Steuerung Modul (110) die Abgabe der Ultraschallenergie beendet, wenn die Geschwindigkeit der Vorrichtung zur Behandlung (100) größer als ein oberer Schwellenwert ist.

8. Die Vorrichtung zur Behandlung (100) nach dem vorhergehenden Anspruch 1, wobei ferner die Ultraschallenergiequelle in der Art und Weise konfiguriert ist, dass sie die Ultraschallenergie durch ein akustisches Fenster mit einer Dicke von etwa einer halben Wellenlänge der Ultraschallenergie oder etwa einem einfachen Vielfachen einer halben Wellenlänge der Ultraschallenergie abgibt.

9. Die Vorrichtung zur Behandlung (100) nach dem vorhergehenden Anspruch 8, wobei ferner das Steuerung Modul (110) in der Art und Weise konfiguriert ist, dass das Steuerung Modul (110) ferner ein Beendigungssignal bereitstellt, um die Abgabe der Ultraschallenergie zu beenden, wenn festgestellt wird, dass die Ultraschallenergie durch das akustische Fenster reflektiert wird, wenn die Ultraschallenergiequelle von dem interessierenden Bereich abgekoppelt ist.

10. Die Vorrichtung zur Behandlung (100) nach dem vorhergehenden Anspruch 1, wobei ferner die Vorrichtung zur Behandlung (100) ein mit dem Gehäuse (112) gekoppeltes Rollelement (115) umfasst, und wobei ferner die Vorrichtung zur Behandlung (100) relativ zu der Zieloberfläche (106) durch ein Drehen des Rollelements (115) entlang der Zieloberfläche (106) in einer Drehrichtung beweglich ist, während die Kopplung zwischen der Ultraschallenergiequelle und dem interessierenden Bereich beibehalten wird.

11. Die Vorrichtung zur Behandlung (100) nach dem vorhergehenden Anspruch 10, wobei ferner das Rollelement (115) außerhalb eines Emissionspfades der Ultraschallenergie von der Ultraschallenergiequelle angeordnet ist.

## Revendications

1. Dispositif de traitement (100) destiné à transmettre une énergie ultrasonore (170) dans une région d'intérêt (108) sur ou au-dessous d'une surface cible (106), le dispositif de traitement (100) comprenant :
un boîtier (112) ;
un module de transducteur (150) couplé au boîtier (112), le module de transducteur (150) comprenant une source d'énergie ultrasonore (160) configurée pour transmettre l'énergie ultrasonore, la transmission de l'énergie ultrasonore étant pulsée, le module de transducteur (150) présentant une paroi de module de transducteur (151) qui comporte une surface incurvée présentant un rayon de courbature non nul, le dispositif de traitement (100) étant mobile par rapport à la surface cible (106) tout en conservant le contact entre la surface incurvée et la surface cible (106),
un ou plusieurs capteurs de position (140) en communication avec un module de commande (110) et configurés pour émettre un signal de position représentant une position du dispositif de traitement (100) par rapport à la surface cible (106) ; et
le module de commande (110) étant configuré pour commander la source d'énergie ultrasonore (160),
le capteur de position (140) étant configuré pour suivre une position du module de transducteur à ultrasons (110) pendant le traitement par rapport à un modèle de déplacement du module de transducteur à ultrasons qui fait partie d'un plan de traitement et calculer une distance parcourue le long de la surface cible (106),
le capteur de position (140) ou le module de commande (110) étant configuré pour déterminer une distance entre des impulsions d'énergie ultrasonore thérapeutique pour créer une pluralité de zones de traitement, et
le capteur de position (140) ou le module de commande (110) étant configuré pour déterminer la distance, indépendamment de la vitesse à laquelle le module de transducteur (150) se déplace, à laquelle une impulsion d'énergie ultrasonore thérapeutique doit être émise.

2. Dispositif de traitement (100) selon la revendication 1, dans lequel le module de transducteur (150) est étanche et contient un milieu de couplage pour faciliter le couplage de la source d'énergie ultrasonore à la région d'intérêt.

3. Dispositif de traitement (100) selon la revendication 1, le dispositif de traitement (100) comprenant en outre un capteur de contact (144) en communication avec le module de commande (110) et configuré pour déterminer quand la source d'énergie ultrasonore est couplée acoustiquement à la région d'intérêt.

4. Dispositif de traitement (100) selon la revendication 3, dans lequel le module de commande (110) est configuré pour fournir le signal d'arrêt qui met fin à la transmission de l'énergie ultrasonore si le capteur de contact (144) détermine que la source d'énergie ultrasonore n'est pas couplée à la région d'intérêt.

5. Dispositif de traitement (100) selon la revendication 1, dans lequel le module de commande (110) est configuré pour fournir le signal d'arrêt qui met fin à la transmission de l'énergie ultrasonore si la position du dispositif de traitement (100) est en dehors d'une zone de traitement prédéfinie.

6. Dispositif de traitement (100) selon la revendication 1, le dispositif (100) comprenant en outre un élément roulant (115) et un axe (124) positionné à travers un axe central de l'élément roulant (115).

7. Dispositif de traitement (100) selon la revendication 1, dans lequel le module de commande (110) est configuré pour mettre fin à la transmission de l'énergie ultrasonore si la vitesse du dispositif de traitement (100) dépasse un seuil supérieur.

8. Dispositif de traitement (100) selon la revendication 1, dans lequel la source d'énergie ultrasonore est configurée pour transmettre l'énergie ultrasonore à travers une fenêtre acoustique présentant une épaisseur d'environ une demi-longueur d'onde de l'énergie ultrasonore ou d'environ un multiple simple d'une demi-longueur d'onde de l'énergie ultrasonore.

9. Dispositif de traitement (100) selon la revendication 8, dans lequel le module de commande est configuré pour fournir un signal d'arrêt pour mettre fin à la transmission de l'énergie ultrasonore s'il est déterminé que l'énergie ultrasonore est réfléchie par la fenêtre acoustique quand la source d'énergie ultrasonore est découplée de la région d'intérêt.

10. Dispositif de traitement (100) selon la revendication 1, le dispositif de traitement (100) comprenant en outre un élément roulant (115) couplé au boîtier (112), le dispositif de traitement (100) étant mobile par rapport à la surface cible (106) en faisant tourner l'élément roulant (115) le long de la surface cible (106) dans un sens de rotation tout en conservant le couplage entre la source d'énergie ultrasonore et la région d'intérêt.

11. Dispositif de traitement (100) selon la revendication 10, dans lequel l'élément roulant (115) est disposé en dehors d'un trajet d'émission de l'énergie ultrasonore provenant de la source d'énergie ultrasonore.
